# EUROPEAN PATENT APPLICATION

(11) **EP 4 817 549 A2**
(43) Date of publication of application: **07.10.2026**
(21) Application number: 26185553.0
(22) Date of filing: 13.07.2022
(51) Int. Cl.: A61B 34/20

(54) **BIOSIGNAL-BASED INTRACARDIAC NAVIGATION SYSTEMS, DEVICES, COMPONENTS AND METHODS**

(30) Priority: 13.07.2021 US 202163221291 P; 15.07.2021 US 202163222346 P; 02.06.2022 US 202217831249
(62) Document number(s) of the earlier application(s) in accordance with Art. 76 EPC: 22184693.4 / 4 119 054
(71) Applicant: Boston Scientific Scimed, Inc., Maple Grove, Minnesota 55311 (US)
(72) Inventor: Denner, Stefan, 80333 München (DE); Grund, Alessa Tabea, 81679 München (DE)
(74) Representative: Pfenning, Meinig & Partner mbB

(57) **Abstract**

In some embodiments, there are provided systems, devices, components, and corresponding methods configured to permit navigation and or positioning of an intra-cardiac electrophysiological (EP) mapping basket of an EP mapping catheter inside or near an atrium or other heart chamber of a patient's heart using biosignals or intra-cardiac signals. In one embodiment, QRS complexes are extracted or isolated from intra-cardiac signals sensed by electrodes mounted on the EP mapping basket. Using the QRS complexes, one or more computing devices then determine the locations of the electrodes inside or near the patient's atrium that are associated with each isolated or extracted QRS complex. The one or more computing devices can also be used to determine changes in the three-dimensional locations and orientations of the basket and the electrodes thereof as the basket is moved around, in, or near the patient's atrium, heart chamber, or other portion of the patient's heart. The computing device can also be configured to use position change detection methods to detect basket catheter movements inside or near the patient's heart, determine the three-dimensional coordinates ***X*** of each electrode for a given position and orientation of the basket within the patient's heart, determine and display to a user multiple positions of the basket inside or near the patient's heart, and provide a visual display to a user of the locations of the electrodes and/or basket inside or near the patient's heart.

## Description

### Field of the Invention

Various embodiments described and disclosed herein relate to the field of medicine generally, and more particularly to detecting, diagnosing, predicting and treating cardiac rhythm disorders such as atrial fibrillation in a patient's heart.

### Background

Persistent atrial fibrillation (AF) is assumed to be caused by structural changes in atrial tissue, which can manifest themselves as multiwavelet re-entry and/or stable rotor mechanisms (see, e.g., De Groot MS et al., "Electropathological Substrate of Longstanding Persistent Atrial Fibrillation in Patients with Structural Heart Disease Epicardial Breakthrough," Circulation, 2010, 3: 1674-1682). Radio frequency (RF) ablation targeting such host drivers of AF is generally accepted as the best therapeutic approach. RF ablation success rates in treating AF cases are currently limited, however, by a lack of diagnostic tools that are capable of precisely determining the source (or type), and location, of such AF drivers. Better diagnostic tools would help reduce the frequency and extent of cardiac ablation procedures to the minimum amount required to treat AF, and would help balance the benefits of decreased fibrillatory burden against the morbidity of increased lesion load.

One method currently employed to localize AF drivers is the TOPERA^{®} RhythmView^{®} system, which employs a basket catheter having 64 electrodes arranged in an 8 x 8 pattern from which the system records unipolar electrograms or electrogram signals (EGMs). The RhythmView^{®} algorithm creates a propagation map of the 64 electrodes through a phase analysis of EGM peaks after improving the signal to noise ratio through filtering and subtraction of a simulated compound ECG artifact. The RhythmView^{®} algorithm detects where peak sequences between electrodes show a circular pattern candidate for a re-entry cycle and indicates those locations in a Focal Impulse and Rotor Map (FIRM) using A1 to H8 chess field coordinates for the electrodes. The resolution of the TOPERA system is limited by the spacing of the electrodes and consequently does not show the details of the AF drivers. In particular, the TOPERA system cannot show if a circular EGM wavefront is actively generated by a re-entry mechanism and is therefore is a driver of AF (i.e., an active rotor), or whether a circular EGM wavefront simply represents turbulence passively generated by an EGM wavefront hitting a barrier (i.e., a passive rotor). In addition, the TOPERA system does not show the direction of AF wavefront propagation, and does not provide the spatial or temporal resolution required to detect singularities associated with the generation of an active rotor.

A recent independent multicenter study ("OASIS, Impact of Rotor Ablation in Non-Paroxysmal AF Patients: Results from a Randomized Trial," Sanghamitra Mohanty, et al. and Andrea Natale, J Am Coll Cardiol. 2016) reported that the results obtained using TOPERA FIRM technology were inferior to those provided by non-specific ablation of the posterior wall of the left atrium. Moreover, the results suggested that FIRM based ablation is not sufficient for therapeutic success without pulmonary vein isolation (PVI) being performed in parallel. Although there are some questions about the methodology of this trial, many experts are convinced that the resolution and interpretability of the TOPERA system need to be improved.

In another approach to the problem, Toronto scientists recently presented a strategy to analyze EGM wave propagation using "Omnipolar Mapping," which seeks to measure beat-by-beat conduction velocity and direction (see, e.g., "Novel Strategy for Improved Substrate Mapping of the Atria: Omnipolar Catheter and Signal Processing Technology Assesses Electrogram Signals Along Physiologic and Anatomic Directions," D. Curtis Deno et al. and Kumaraswamy Nanthakumar; Circulation. 2015;132:A19778). This approach starts with the time derivative of a unipolar EGM as measured by a set of electrodes having known distances to one other. Assuming constant velocity, the velocity and direction representing the best fit for a spatial derivative of the measured EGM are calculated and used to represent an estimate of the E field. According to a communication by Dr. Nanthakumar at the 2016 CardioStim Convention in Nice, France, however, this method remains incapable of dealing successfully with complex data sets, such as those obtained during an episode of AF.

AF is the most common supraventricular tachyarrhythmia worldwide and is associated with a significant health burden. Catheter ablation of pulmonary veins (PV) has been established as a therapeutic option for patients with symptomatic drug-refractory paroxysmal AF and results in high clinical success. However, the treatment of persistent and long-standing persistent AF is still challenging. A large number of patients present with recurrence of atrial tachyarrhythmia during mid- and long-term follow up. To achieve higher success rates, different ablation strategies have been reported, such as targeting additional AF sources. The initial results of focal impulse and rotor (FIRM) mapping for guiding catheter ablation of AF seemed to be promising. However, currently available systems for AF driver identification still have significant limitations, such as limited spatial resolution and difficulties in discriminating between active and passive rotors.

AF is a heart disease that prevents the upper chambers of the heart (the atria) from contracting properly, which can result in the heart quivering instead of beating in a correct rhythm. A diagnosis of AF can result in patient having a five-fold risk of stroke, a three-fold incidence of congestive heart failure, and higher mortality. For effective treatment of AF, the electrical activity of the atria needs to be understood, which can be accomplished by placing a catheter with attached electrodes inside the atria, recording the resulting intra-cardiac signals as electrograms (or EGMs), and then viewing or otherwise analyzing the resulting EGMs. To better sense and understand electrical activity in the atria, multiple EGMs with the catheter in multiple positions are generally recorded. For the placement and navigation of the catheter, 3D catheter navigation systems are typically employed, which permit accurate visualization and determination of the position of the catheter inside the atria. Unfortunately, such navigation systems have the shortcomings of either requiring additional hardware and/or introducing artifacts into the EGMs, not to mention the significant costs and technical complications associated with such navigation systems. Artifacts in EGMs can be especially critical, since EGMs are the foundation upon which treatment decisions are made. EGMs recorded with high fidelity, and not containing or containing few artifacts can be crucial to accurate diagnosis and subsequent treatment success.

What is needed are improved means and methods of acquiring and processing intracardiac electrogram signals that reliably and accurately yield the precise locations and sources of cardiac rhythm disorders in a patient's heart. Doing so would enable cardiac ablation procedures to be carried out with greater locational precision, and would result in higher rates of success in treating cardiac rhythm disorders such as AF.

### Summary

In one embodiment, there is provided a method of determining at least one of a position and an orientation of an intra-cardiac electrophysiological (EP) mapping basket of an EP mapping catheter when the basket is disposed inside or near an atrium or other heart chamber or portion of a patient's heart, the EP mapping basket comprising a plurality of electrodes mounted on one or more arms or splines thereof, each electrode having a location or position on one or more splines or arms associated therewith. The method comprises recording or acquiring a plurality of intra-cardiac signals inside or near the heart of the patient using the plurality of electrodes, a data acquisition or recording amplifier or device, and a computing device, the data acquisition or recording amplifier or device, and the computing device, being operably connected to the EP mapping catheter and the electrodes thereof, each of the recorded or acquired intra-cardiac signals having at least one electrode associated therewith; using the computing device, isolating or extracting ventricular signals from the at least some of the recorded or acquired intra-cardiac signals; using the computing device, determining, using the extracted or isolated ventricular signals and the computing device, the locations of the electrodes inside or near the patient's atrium, heart chamber or other portion of the patient's heart that are associated with each isolated or extracted ventricular signal.

The foregoing method can further comprise one or more of the following: (i) navigating the intra-cardiac EP mapping basket of the EP mapping catheter inside or near the atrium or other heart chamber or portion of the patient's heart using the determined locations of the electrodes; (ii) wherein the isolated or extracted ventricular signals comprise at least one of QRS and QRST signals; (iii) recording or acquiring at least one body surface signal from the patient using at least one body surface electrode, the at least one body surface signal being acquired or recorded simultaneously or substantially simultaneously with the recorded or acquired intra-cardiac signals; (iv) using the computing device, isolating or extracting at least one ventricular signal from the recorded or acquired body surface signal, and determining, using the extracted or isolated intra-cardiac and at least one body surface ventricular signals and the computing device, the locations of the electrodes inside the patient's heart that are associated with each isolated or extracted ventricular signal; (v) wherein the isolated or extracted ventricular signals comprise QRS complexes; (vi) moving the basket inside the patient's atrium to different positions, and recording or acquiring intra-cardiac signals at each such position; (vii) using the computing device, removing low-frequency or other artefacts or noise from the recorded or acquired intra-cardiac and/or body surface signals; (viii) recording or acquiring a plurality of intra-cardiac signals for each electrode while the basket is in a given position within the patient's atrium, and, using the computing device, isolating or extracting a ventricular signal for each such intra-cardiac signal.; (ix) using the computing device, for each electrode an averaged ventricular signal is generated using the plurality of intra-cardiac signals; (x) wherein the averaged ventricular signal is an averaged QRS complex; (xi) using the computing device, determining changes in the three-dimensional locations and orientations of the basket and the electrodes thereof as the basket is moved around, in or near the patient's atrium; (xii) using the computing device, using position change detection methods in the computing device to detect basket catheter movements; (xiii) using the computing device, determining the three-dimensional coordinates ***X*** of each electrode for a given position and orientation of the basket within the patient's atrium; (xiv) using the computing device, embedding multiple QRS complexes associated with the ventricular signals acquired or recorded at multiple positions into an embedding space such that multiple positions of the basket at different locations and/or orientations in or near the patient's atrium can be visualized by a user on a screen or monitor operably connected to the computing device; (xv) wherein the multiple positions of the basket can be employed by the user to navigate the basket inside or near the patient's atrium; (xvi) providing a visual display to a user of the locations of the electrodes and/or basket inside or near the patient's atrium; (xvii) wherein an autoencoder is employed in the computing device to compress QRS complexes into low-dimensional representations thereof; (xviii) wherein the computing device is configured to learn relative positioning of QRS complexes in the embedding space; (xix) using the computing device and the isolated or extracted ventricular signals, at least one artificial neural network to determine the locations of the electrodes and/or basket inside or near the patient's atrium; (xx) wherein the at least one artificial neural network further comprises at least one feed forward network; (xxi) wherein the at least one artificial neural network further comprises at least one convolutional neural network; (xxii) wherein the at least one artificial neural network further comprises at least one autoencoder; and (xxiii) wherein the locations of the electrodes and/or basket inside or near the patient's atrium are provided in a visualization to a user in real time or near real time.

In another embodiment, there is provided a system configured to determine at least one of a position and an orientation of an intra-cardiac electrophysiological (EP) mapping basket of an EP mapping catheter when the basket is disposed inside or near an atrium or other heart chamber or portion of a patient's heart, the EP mapping basket comprising a plurality of electrodes mounted on one or more arms or splines thereof, each electrode having a location or position on one or more splines or arms associated therewith. The system comprises: (a) at least one computing device; (b) at least one data acquisition device operably connected to the at least one computing device or configured to provide as outputs therefrom at least one of electrical signals and at least one body surface electrogram signal; and (c) a display or monitor operably connected to the at least one computing device and configured to visually display to a user results generated by the at least one computing device; wherein the computing device comprises at least one non-transitory computer readable medium configured to store instructions executable by at least one processor to extract atrial signals from the plurality of electrical signals, the computing device being configured to: (i) receive or acquire, using the data acquisition device, the plurality of electrical signals using electrodes and at least one body surface electrogram signal using the at least one body surface electrode located on one or more body surfaces of the patient, wherein the electrical signals and the at least one body surface electrogram signal are acquired over the same or substantially the same first time window, the first time window being sufficiently long to capture a plurality of QRS complexes; (ii) recording or acquiring a plurality of intra-cardiac signals inside or near the heart of the patient using the plurality of electrodes, the data acquisition device, and the computing device, the data acquisition device and the computing device being operably connected to the EP mapping catheter and the electrodes thereof, each of the recorded or acquired intra-cardiac signals having at least one electrode associated therewith; (iii) using the computing device, isolating or extracting ventricular signals from the at least some of the recorded or acquired intra-cardiac signals; and (iv) using the computing device, determining, using the extracted or isolated ventricular signals and the computing device, the locations of the electrodes inside or near the patient's atrium, heart chamber or other portion of the patient's heart that are associated with each isolated or extracted ventricular signal.

In the foregoing system, one or more of the following may be included in the system: (i) configuring the system to navigate the intra-cardiac EP mapping basket of the EP mapping catheter inside or near the atrium or other heart chamber or portion of the patient's heart using the determined locations of the electrodes; (ii) wherein the isolated or extracted ventricular signals comprise at least one of QRS and QRST signals; (iii) wherein the isolated or extracted ventricular signals comprise QRS complexes; (iv) wherein for each electrode an averaged ventricular signal is generated using the plurality of intra-cardiac signals; (v) wherein the averaged ventricular signal is an averaged QRS complex; (vi) configuring the computing device to determine changes in the three-dimensional locations and orientations of the basket and the electrodes thereof as the basket is moved around, in or near the patient's atrium; (vii) configuring the computing device to use position change detection methods to detect basket catheter movements; (viii) configuring the computing device to determine three-dimensional coordinates ***X*** of each electrode for a given position and orientation of the basket within the patient's atrium; (ix) configuring the computing device to embed multiple QRS complexes associated with the ventricular signals acquired or recorded at multiple positions into an embedding space such that multiple positions of the basket at different locations and/or orientations in or near the patient's atrium can be visualized by a user on the screen or monitor; (x) wherein multiple positions of the basket can be employed by the user to navigate the basket inside or near the patient's atrium; (xi) the monitor or screen being configured to provide a visual display to the user of the locations of at least one of the electrodes and the basket inside or near the patient's atrium; (xii) wherein an autoencoder is employed in the computing device to compress QRS complexes into low-dimensional representations thereof; (xiii) wherein the computing device is configured to learn relative positioning of QRS complexes in the embedding space; (xiv) configuring the computing device and the isolated or extracted ventricular signals to employ at least one artificial neural network to determine the locations of at least one of the electrodes and the basket inside or near the patient's atrium; (xv) wherein the at least one artificial neural network further comprises at least one feed forward network; (xvi) wherein the at least one artificial neural network further comprises at least one convolutional neural network; (xvii) wherein the at least one artificial neural network further comprises at least one autoencoder; and (xviii) wherein the locations of the electrodes and/or basket inside or near the patient's atrium are provided in a visualization to a user in real time or near real time.

Further embodiments are disclosed herein or will become apparent to those skilled in the art after having read and understood the claims, specification and drawings hereof.

### Brief Description of the Drawings

The patent or application file contains at least one drawing executed in color. Copies of this patent or patent application publication with color drawing(s) will be provided by the Office upon request and payment of the necessary fee.

Different aspects of the various embodiments will become apparent from the following specification, drawings and claims in which:
Fig. 1A shows one embodiment of a combined cardiac electrophysiological mapping (EP), pacing and ablation system 100;
Fig. 1B shows one embodiment of a computer system 300;
Fig. 2A shows one embodiment of an intra-cardiac imaging and/or navigation system 100;
Fig. 2B shows one embodiment of a body surface imaging and/or navigation system;
Fig. 3A shows a visualization of blood flow in the human heart;
Fig. 3B shows a typical human heart transmembrane voltage depicted over time;
Fig. 3C shows a schematic representation of a cross-section view of the human heart;
Fig. 4A shows a schematic view of a typical ECG;
Fig. 4B shows an example of a unipolar EGM;
Fig. 4C shows an example of a basket catheter;
Fig. 5 shows one example of One example of a convolution operation;
Fig. 6 shows an example of results of simultaneous recording of intracardiac EGMs and body surface ECGs;
Fig. 7A shows an example of a screenshot from a real-time navigation system;
Fig. 7B shows another example of a screenshot from a real-time navigation system;
Fig. 8 shows frontal, horizontal and sagittal representations, according to columns arranged left to right, of ventricular depolarizations;
Fig. 9 shows a series of extracted QRS-complexes for ~60 second EGM recordings;
Fig. 10A shows one example of ventricular isolation;
Fig. 10B shows N QRS-complexes extracted from a single location x;
Fig. 10C shows N averaged QRS-complexes from 64 electrodes of a basket catheter;
Fig. 11 shows a distance matrix for an undeformed spherical basket in a basket catheter;
Fig 12 shows one embodiment of an autoencoder configured to learn the manifold of QRS-complexes;
Fig. 13 shows an example of a weighting matrix M for loss *L_{z}*;
Fig. 14 shows one embodiment of a computation graph and flow diagram for *L_{z}*;
Fig. 15 shows Fig. 15 shows one embodiment of a Confusion Matrix, and how TP, FN, FP and TN can be defined;
Fig. 16A an ROC Curve for an example training set, where along the x-axis the FPR is displayed, and along the y-axis the TPR is displayed;
Fig. 16B shows different thresholds are analyzed resulting in a maximal *TPR - FPR* score;
Fig. 16C shows a histogram of identity correspondences, where a margin between same position and position change identity correspondences is displayed;
Fig. 17A illustrates one embodiment of atrial collapse in relative positioning;
Fig. 17B illustrates another embodiment of atrial collapse in relative positioning;
Fig. 17C shows an example of minimal distance error D achieved with a *β* of 1, where the x-axis depicts different choices of beta, and the y-axis depicts the average distance error of the estimates against a ground truth of a training or validation set;
Figs. 17D-17G show examples of qualitative comparisons of ground truth reconstructions against estimates, where Figs. 17D and 17E show examples corresponding to the right atrium, and where Figs. 17F and 17G show examples corresponding to the left atrium;
Figs. 18A and 18B show correspondence matches that are reasonable and unreasonable, respectively;
Figs. 19A and 19B show examples of correct and incorrect windings of a basket catheter, respectively, and
Fig. 20 shows one embodiment of a one method for determining the locations of electrodes inside a patient's heart using biosignals.

The drawings are not necessarily to scale. Like numbers refer to like parts or steps throughout the drawings.

### Detailed Descriptions of Some Embodiments

Described herein are various embodiments of systems, devices, components and methods for diagnosing and treating cardiac rhythm disorders in a patient's heart using electrophysiological mapping or electrographic flow (EGF) techniques, as well as imaging, navigation, cardiac ablation and other types of medical systems, devices, components, and methods. Various embodiments described and disclosed herein also relate to systems, devices, components and methods for discovering with enhanced precision the location(s) of the source(s) of different types of cardiac rhythm disorders and irregularities. Such cardiac rhythm disorders and irregularities, include, but are not limited to, arrhythmias, atrial fibrillation (AF or A-fib), atrial tachycardia, atrial flutter, paroxysmal fibrillation, paroxysmal flutter, persistent fibrillation, ventricular fibrillation (V-fib), ventricular tachycardia, atrial tachycardia (A-tach), ventricular tachycardia (V-tach), supraventricular tachycardia (SVT), paroxysmal supraventricular tachycardia (PSVT), Wolff-Parkinson-White syndrome, bradycardia, sinus bradycardia, ectopic atrial bradycardia, junctional bradycardia, heart blocks, atrioventricular block, idioventricular rhythm, areas of fibrosis, breakthrough points, focus points, re-entry points, premature atrial contractions (PACs), premature ventricular contractions (PVCs), and other types of cardiac rhythm disorders and irregularities.

Various embodiments of EGF techniques, methods, systems, devices, and components are described and disclosed herein, which involve the acquisition of intra-cardiac and/or body surface electrograms, and the subsequent processing and analysis of such electrograms to reveal the locations of sources of cardiac rhythm disorders in a patient's heart, such as rotors and sources that cause or contribute to AF. That is, many of the various techniques, methods, systems, devices, and components described and disclosed herein may be referred to collectively as pertaining to "EGF."

Systems and methods configured to detect in a patient's heart a location of a source of at least one cardiac rhythm disorder are disclosed herein. In the following description, for the purposes of explanation, numerous specific details are set forth in order to provide a thorough understanding of example embodiments or aspects. It will be evident, however, to one skilled in the art that an example embodiment may be practiced without necessarily using all of the disclosed specific details.

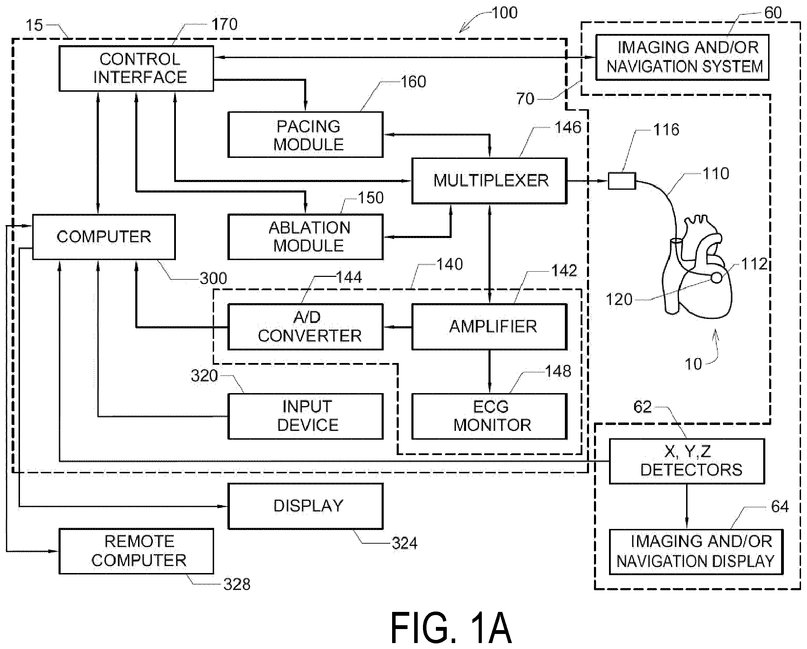

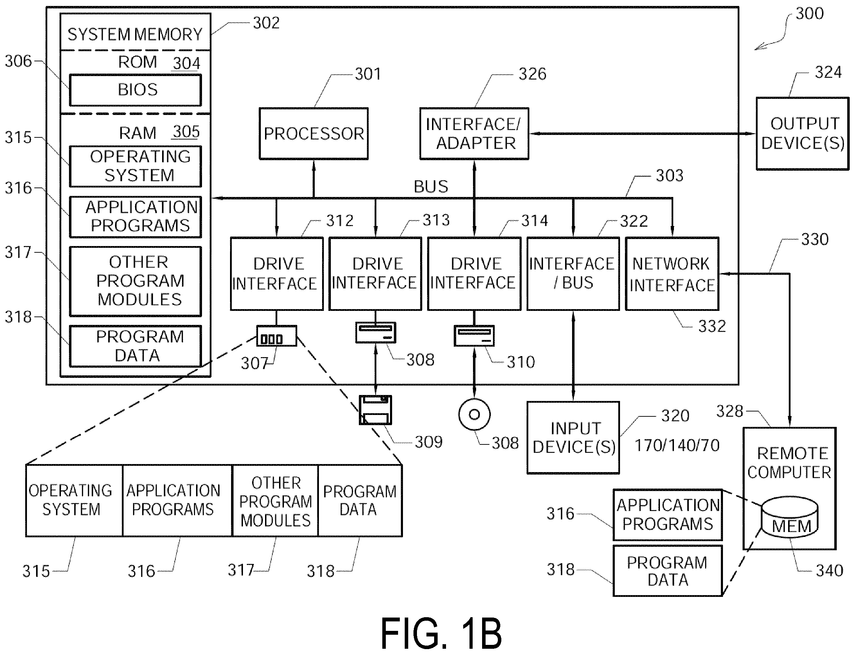

Referring now to Fig. 1A, there is illustrated one embodiment of a combined cardiac electrophysiological mapping (EP), pacing and ablation system 100. Note that in some embodiments system 100 may not include ablation module 150 and/or pacing module 160. Among other things, the embodiment of system 100 shown in Fig. 1B is configured to detect and reconstruct cardiac activation information acquired from a patient's heart relating to cardiac rhythm disorders and/or irregularities, and is further configured to detect and discover the location of the source of such cardiac rhythm disorders and/or irregularities with enhanced precision relative to prior art techniques. In some embodiments, system 100 is further configured to treat the location of the source of the cardiac rhythm disorder or irregularity, for example by ablating the patient's heart at the detected location.

The embodiment of system 100 shown in Fig. 1B comprises five main functional units: electrophysiological mapping (EP mapping unit) 140 (which is also referred to herein as data acquisition device 140), ablation module 150, pacing module 160, imaging and/or navigation system 70, and computer or computing device 300. Data acquisition, processing and control system 15 comprises data acquisition device 140, ablation module 150, pacing module 160, control interface 170 and computer or computing device 300. In one embodiment, at least one computer or computing device or system 300 is employed to control the operation of one or more of systems, modules and devices 140, 150, 160, 170 and 70. Alternatively, the respective operations of systems, modules or devices 140, 150, 160, 170 and 70 may be controlled separately by each of such systems, modules and devices, or by some combination of such systems, modules and devices.

Instead of being operably connected (e.g., through Bluetooth signals, a LAN or WAN network, or through the cloud), or directly connected, to computing device 300, data acquisition device 140 may be configured to provide as outputs therefrom saved or stored body surface electrogram signals, which can be, by way of example, saved or stored on a hard drive, in a memory, on a USB stick, or other suitable storage device, and where the saved or stored body surface electrogram signals are later or subsequently provided as inputs to computing device 300 for processing and analysis.

Computer or computing device 300 may be configured to receive operator inputs from an input device 320 such as a keyboard, mouse and/or control panel. Outputs from computer 300 may be displayed on display or monitor 324 or other output devices (not shown in Fig. 1B). Computer 300 may also be operably connected to a remote computer or analytic database or server 328. At least each of components, devices, modules and systems 60, 110, 140, 146, 148, 150, 170, 300, 324 and 328 may be operably connected to other components or devices by wireless (e.g., Bluetooth) or wired means. Data may be transferred between components, devices, modules or systems through hardwiring, by wireless means, or by using portable memory devices such as USB memory sticks.

During electrophysiological (EP) mapping procedures, multi-electrode catheter 110 is typically introduced percutaneously into the patient's heart 10. Catheter 110 is passed through a blood vessel (not shown), such as a femoral vein or the aorta, and thence into an endocardial site such as the atrium or ventricle of the heart 10.

It is contemplated that other catheters, including other types of mapping or EP catheters, lasso catheters, pulmonary vein isolation (PVI) ablation catheters (which can operate in conjunction with sensing lasso catheters), ablation catheters, navigation catheters, and other types of EP mapping catheters such as EP monitoring catheters and spiral catheters may also be introduced into the heart, and that additional surface electrodes may be attached to the skin of the patient to record electrocardiograms (ECGs).

When system 100 is operating in an EP mapping mode, multi-electrode catheter 110 functions as a detector of intra-electrocardiac signals, while optional surface electrodes may serve as detectors of surface ECGs. In one embodiment, the analog signals obtained from the intracardiac and/or surface electrodes are routed by multiplexer 146 to data acquisition device 140, which comprises an amplifier 142 and an A/D converter (ADC) 144. The amplified or conditioned electrogram signals may be displayed by electrocardiogram (ECG) monitor 148. The analog signals are also digitized via ADC 144 and input into computer 300 for data processing, analysis and graphical display.

In one embodiment, catheter 110 is configured to detect cardiac activation information in the patient's heart 10, and to transmit the detected cardiac activation information to data acquisition device 140, either via a wireless or wired connection. In one embodiment that is not intended to be limiting with respect to the number, arrangement, configuration, or types of electrodes, catheter 110 includes a plurality of 64 electrodes, probes and/or sensors A1 through H8 arranged in an 8x8 grid that are included in electrode mapping assembly 120, which is configured for insertion into the patient's heart through the patient's blood vessels and/or veins. Other numbers, arrangements, configurations and types of electrodes in catheter 110 are, however, also contemplated. In most of the various embodiments, at least some electrodes, probes and/or sensors included in catheter 110 are configured to detect cardiac activation or electrical signals, and to generate electrocardiograms or electrogram signals, which are then relayed by electrical conductors from or near the distal end 112 of catheter 110 to proximal end 116 of catheter 110 to data acquisition device 140.

Note that in some embodiments of system 100, multiplexer 146 is not employed for various reasons, such as sufficient electrical conductors being provided in catheter 110 for all electrode channels, or other hardware design considerations. In other embodiments, multiplexer 146 is incorporated into catheter 110 or into data acquisition device 140. In still further embodiments, multiplexer 146 is optional or not provided at all, and data acquisition device 140, ablation module 150, and/or pacing module 160 are employed separately and/or operate independently from one another. In addition, in some embodiments computing device 300 may be combined or integrated with one or more of data acquisition device 140, ablation module 150, and/or pacing module 160.

In one embodiment, a medical practitioner or health care professional employs catheter 110 as a roving catheter to locate the site of the location of the source of a cardiac rhythm disorder or irregularity in the endocardium quickly and accurately, without the need for open-chest and open-heart surgery. In one embodiment, this is accomplished by using multi-electrode catheter 110 in combination with real-time or near-real-time data processing and interactive display by computer 300, and optionally in combination with imaging and/or navigation system 70. In one embodiment, multi-electrode catheter 110 deploys at least a two-dimensional array of electrodes against a site of the endocardium at a location that is to be mapped, such as through the use of a Biosense Webster^{®} PENTARAY^{®} EP mapping catheter . The intracardiac or electrogram signals detected by the catheter's electrodes provide data sampling of the electrical activity in the local site spanned by the array of electrodes.

In one embodiment, the electrogram signal data are processed by computer 300 to produce a display showing the locations(s) of the source(s) of cardiac rhythm disorders and/or irregularities in the patient's heart 10 in real-time or near-real-time, further details of which are provided below. That is, at and between the sampled locations of the patient's endocardium, computer 300 may be configured to compute and display in real-time or near-real-time an estimated, detected and/or determined location(s) of the site(s), source(s) or origin)s) of the cardiac rhythm disorder(s) and/or irregularity(s) within the patient's heart 10. This permits a medical practitioner to move interactively and quickly the electrodes of catheter 110 towards the location of the source of the cardiac rhythm disorder or irregularity.

In some embodiments of system 100, one or more electrodes, sensors or probes detect cardiac activation from the surface of the patient's body as surface ECGs, or remotely without contacting the patient's body (e.g., using magnetocardiograms). In another example, some electrodes, sensors or probes may derive cardiac activation information from echocardiograms. In various embodiments of system 100, external or surface electrodes, sensors and/or probes can be used separately or in different combinations, and further may also be used in combination with intracardiac electrodes, sensors and/or probes inserted within the patient's heart 10. Many different permutations and combinations of the various components of system 100 are contemplated having, for example, reduced, additional or different numbers of electrical sensing and other types of electrodes, sensors and/or transducers.

Continuing to refer to Fig. 1B, EP mapping system or data acquisition device 140 is configured to condition the analog electrogram signals delivered by catheter 110 from electrodes A1 through H8 in amplifier 142. Conditioning of the analog electrogram signals received by amplifier 142 may include, but is not limited to, low-pass filtering, high-pass filtering, bandpass filtering, and notch filtering. The conditioned analog signals are then digitized in analog-to-digital converter (ADC) 144. ADC 144 may further include a digital signal processor (DSP) or other type of processor which is configure to further process the digitized electrogram signals (e.g., low-pass filter, high-pass filter, bandpass filter, notch filter, automatic gain control, amplitude adjustment or normalization, artifact removal, etc.) before they are transferred to computer or computing device 300 for further processing and analysis.

As discussed above, in some embodiments, multiplexer 146 is separate from catheter 110 and data acquisition device 140, and in other embodiments multiplexer 146 is combined in catheter 110 or data acquisition device 140.

In some embodiments, the rate at which individual electrogram and/or ECG signals are sampled and acquired by system 100 can range between about 0.25 milliseconds and about 8 milliseconds, and may be about 0.5 milliseconds, about 1 millisecond, about 2 milliseconds or about 4 milliseconds. Other sample rates are also contemplated. While in some embodiments system 100 is configured to provide unipolar signals, in other embodiments system 100 is configured to provide bipolar signals.

In one embodiment, system 100 can include a BARD^{®} LABSYSTEM^{™} PRO EP Recording System, which is a computer and software driven data acquisition and analysis tool designed to facilitate the gathering, display, analysis, pacing, mapping, and storage of intracardiac EP data. Also in one embodiment, data acquisition device 140 can include a BARD^{®} CLEARSIGN^{™} amplifier, which is configured to amplify and condition electrocardiographic signals of biologic origin and pressure transducer input, and transmit such information to a host computer (e.g., computer 300 or another computer).

As shown in Fig. 1B, and as described above, in some embodiments system 100 includes ablation module 150, which may be configured to deliver RF ablation energy through catheter 110 and corresponding ablation electrodes disposed near distal end 112 thereof, and/or to deliver RF ablation energy through a different catheter (not shown in Fig. 1B). Suitable ablation systems and devices include, but are not limited to, cryogenic ablation devices and/or systems, radiofrequency ablation devices and/or systems, ultrasound ablation devices and/or systems, high-intensity focused ultrasound (HIFU) devices and/or systems, chemical ablation devices and/or systems, and laser ablation devices and/or systems.

When system 100 is operating in an optional ablation mode, multi-electrode catheter 110 fitted with ablation electrodes, or a separate ablation catheter, is energized by ablation module 150 under the control of computer 300, control interface 170, and/or another control device or module. For example, an operator may issue a command to ablation module 150 through input device 320 to computer 300. In one embodiment, computer 300 or another device controls ablation module 150 through control interface 170. Control of ablation module 150 can initiate the delivery of a programmed series of electrical energy pulses to the endocardium via catheter 110 (or a separate ablation catheter, not shown in Fig. 1B). One embodiment of an ablation method and device is disclosed in U.S. Patent No. 5,383,917 to Desai et al., the entirety of which is hereby incorporated by reference herein.

In an alternative embodiment, ablation module 150 is not controlled by computer 300, and is operated manually directly under operator control. Similarly, pacing module 160 may also be operated manually directly under operator control. The connections of the various components of system 100 to catheter 110, to auxiliary catheters, or to surface electrodes may also be switched manually or using multiplexer 146 or another device or module.

When system 100 is operating in an optional pacing mode, multi-electrode catheter 110 is energized by pacing module 160 operating under the control of computer 300 or another control device or module. For example, an operator may issue a command through input device 320 such that computer 300 controls pacing module 160 through control interface 170, and multiplexer 146 initiates the delivery of a programmed series of electrical simulating pulses to the endocardium via the catheter 110 or another auxiliary catheter (not shown in Fig. 1B). One embodiment of a pacing module is disclosed in M. E. Josephson et al., in "VENTRICULAR ENDOCARDIAL PACING II, The Role of Pace Mapping to Localize Origin of Ventricular Tachycardia," The American Journal of Cardiology, vol. 50, November 1982.

Computing device or computer 300 is appropriately configured and programmed to receive or access the electrogram signals provided by data acquisition device 140. Computer 300 is further configured to analyze or process such electrogram signals in accordance with the methods, functions and logic disclosed and described herein so as to permit reconstruction of cardiac activation information from the electrogram signals. This, in turn, makes it possible to locate with at least some reasonable degree of precision the location of the source of a heart rhythm disorder or irregularity. Once such a location has been discovered, the source may be eliminated or treated by means that include, but are not limited to, cardiac ablation.

In one embodiment, and as shown in Fig. 1B, system 100 also comprises a physical imaging and/or navigation system 70. Physical imaging and/or navigation device 60 included in system 70 may be, by way of example, a 2- or 3-axis fluoroscope system, an ultrasonic system, a magnetic resonance imaging (MRI) system, a computed tomography (CT) imaging system, and/or an electrical impedance tomography EIT) system. Operation of system 70 be controlled by computer 300 via control interface 170, or by other control means incorporated into or operably connected to imaging or navigation system 70. In one embodiment, computer 300 or another computer triggers physical imaging or navigation system 60 to take "snap-shot" pictures of the heart 10 of a patient (body not shown). A picture image is detected by a detector 62 along each axis of imaging, and can include a silhouette of the heart as well as a display of the inserted catheter 110 and its electrodes A1-H8 (more about which is said below), which is displayed on imaging or navigation display 64. Digitized image or navigation data may be provided to computer 300 for processing and integration into computer graphics that are subsequently displayed on monitor or display 64 and/or 324.

In one embodiment, system 100 further comprises or operates in conjunction with catheter or electrode position transmitting and/or receiving coils or antennas located at or near the distal end of an EP mapping catheter 110, or that of an ablation or navigation catheter 110, which are configured to transmit electromagnetic signals for intra-body navigational and positional purposes.

In one embodiment, imaging or navigation system 70 is used to help identify and determine the precise two- or three-dimensional positions of the various electrodes included in catheter 110 within patient's heart 10, and is configured to provide electrode position data to computer 300. Electrodes, position markers, and/or radio-opaque markers can be located on various potions of catheter 110, mapping electrode assembly 120 and/or distal end 112, or can be configured to act as fiducial markers for imaging or navigation system 70.

Medical navigation systems suitable for use in the various embodiments described and disclosed herein include, but are not limited to, image-based navigation systems, model-based navigation systems, optical navigation systems, electromagnetic navigation systems (e.g., BIOSENSE^{®} WEBSTER^{®} CARTO^{®} system), and impedance-based navigation systems (e.g., the St. Jude^{®} ENSITE^{™} VELOCITY^{™} cardiac mapping system), and systems that combine attributes from different types of imaging AND navigation systems and devices to provide navigation within the human body (e.g., the MEDTRONIC^{®} STEALTHSTATION^{®} system).

In view of the structural and functional descriptions provided herein, those skilled in the art will appreciate that portions of the described devices and methods may be configured as processes, methods, data processing systems, and/or computer methods. Accordingly, these portions of the devices and methods described herein may take the form of a hardware embodiment, a software embodiment, or an embodiment combining software and hardware, such as shown and described with respect to computer system 300 illustrated in Fig. 1B. Furthermore, portions of the devices and methods described herein may be a process or method stored in a computer-usable storage medium having computer readable program code on the medium. Any suitable computer-readable medium may be utilized including, but not limited to, static and dynamic storage devices, hard disks, optical storage devices, and magnetic storage devices.

Certain embodiments of portions of the devices and methods described herein are also described with reference to block diagrams of methods, processes, and systems. It will be understood that such block diagrams, and combinations of blocks diagrams in the Figures, can be implemented using computer-executable instructions. These computer-executable instructions may be provided to one or more processors of a general purpose computer, a special purpose computer, or any other suitable programmable data processing apparatus (or a combination of devices and circuits) to produce a machine, such that the instructions, which executed via the processor(s), implement the functions specified in the block or blocks of the block diagrams.

These computer-executable instructions may also be stored in a computer-readable memory that can direct computer 300 or other programmable data processing apparatus to function in a particular manner, such that the instructions stored in the computer-readable memory result in an article of manufacture including instructions which implement the function specified in an individual block, plurality of blocks, or block diagram. The computer program instructions may also be loaded onto computer 300 or other programmable data processing apparatus to cause a series of operational steps to be performed on the computer or other programmable apparatus to produce a computer implemented process such that the instructions which execute on computer 300 or other programmable apparatus provide steps for implementing the functions specified in an individual block, plurality of blocks, or block diagram.

In this regard, Fig. 1B illustrates only one example of a computer system 300 (which, by way of example, can include multiple computers or computer workstations) that can be employed to execute one or more embodiments of the devices and methods described and disclosed herein, such as devices and methods configured to acquire and process sensor or electrode data, to process image data, and/or transform sensor or electrode data and image data associated with the analysis of cardiac electrical activity and the carrying out of the combined electrophysiological mapping and analysis of the patient's heart 10 and ablation therapy delivered thereto.

Computer system 300 can be implemented on one or more general purpose computer systems or networked computer systems, embedded computer systems, routers, switches, server devices, client devices, various intermediate devices/nodes or standalone computer systems. Additionally, computer system 300 or portions thereof may be implemented on various mobile devices such as, for example, a personal digital assistant (PDA), a laptop computer and the like, provided the mobile device includes sufficient processing capabilities to perform the required functionality.

In one embodiment, computer system 300 includes processing unit 301 (which may comprise a CPU, controller, microcontroller, processor, microprocessor or any other suitable processing device), system memory 302, and system bus 303 that operably connects various system components, including the system memory, to processing unit 301. Multiple processors and other multiprocessor architectures also can be used to form processing unit 301. System bus 303 can comprise any of several types of suitable bus architectures, including a memory bus or memory controller, a peripheral bus, or a local bus. System memory 302 can include read only memory (ROM) 304 and random access memory (RAM) 305. A basic input/output system (BIOS) 306 can be stored in ROM 304 and contain basic routines configured to transfer information and/or data among the various elements within computer system 300.

Computer system 300 can include a hard disk drive 303, a magnetic disk drive 308 (e.g., to read from or write to removable disk 309), or an optical disk drive 310 (e.g., for reading CD-ROM disk 311 or to read from or write to other optical media). Hard disk drive 303, magnetic disk drive 308, and optical disk drive 310 are connected to system bus 303 by a hard disk drive interface 312, a magnetic disk drive interface 313, and an optical drive interface 314, respectively. The drives and their associated computer-readable media are configured to provide nonvolatile storage of data, data structures, and computer-executable instructions for computer system 300. Although the description of computer-readable media above refers to a hard disk, a removable magnetic disk and a CD, other types of media that are readable by a computer, such as magnetic cassettes, flash memory cards, digital video disks and the like, in a variety of forms, may also be used in the operating environment; further, any such media may contain computer-executable instructions for implementing one or more parts of the devices and methods described and disclosed herein.

A number of program modules may be stored in drives and RAM 303, including operating system 315, one or more application programs 316, other program modules 313, and program data 318. The application programs and program data can include functions and methods programmed to acquire, process and display electrical data from one or more sensors, such as shown and described herein. The application programs and program data can include functions and methods programmed and configured to process data acquired from a patient for assessing heart function and/or for determining parameters for delivering a therapy and/or assessing heart function, such as shown and described herein.

A health care provider or other user may enter commands and information into computer system 300 through one or more input devices 320, such as a pointing device (e.g., a mouse, a touch screen, etc.), a keyboard, a microphone, a joystick, a game pad, a scanner, and the like. For example, the user can employ input device 320 to edit or modify the data being input into a data processing method (e.g., only data corresponding to certain time intervals). These and other input devices 320 may be connected to processing unit 301 through a corresponding input device interface or port 322 that is operably coupled to the system bus, but may be connected by other interfaces or ports, such as a parallel port, a serial port, or a universal serial bus (USB). One or more output devices 324 (e.g., display, a monitor, a printer, a projector, or other type of display device) may also be operably connected to system bus 303 via interface 326, such as through a video adapter.

Computer system 300 may operate in a networked environment employing logical connections to one or more remote computers, such as remote computer 328. Remote computer 328 may be a workstation, a computer system, a router, or a network node, and may include connections to many or all the elements described relative to computer system 300. The logical connections, schematically indicated at 330, can include a local area network (LAN) and/or a wide area network (WAN).

When used in a LAN networking environment, computer system 300 can be connected to a local network through a network interface or adapter 332. When used in a WAN networking environment, computer system 300 may include a modem, or may be connected to a communications server on the LAN. The modem, which may be internal or external, can be connected to system bus 303 via an appropriate port interface. In a networked environment, application programs 316 or program data 318 depicted relative to computer system 300, or portions thereof, may be stored in a remote memory storage device 340.

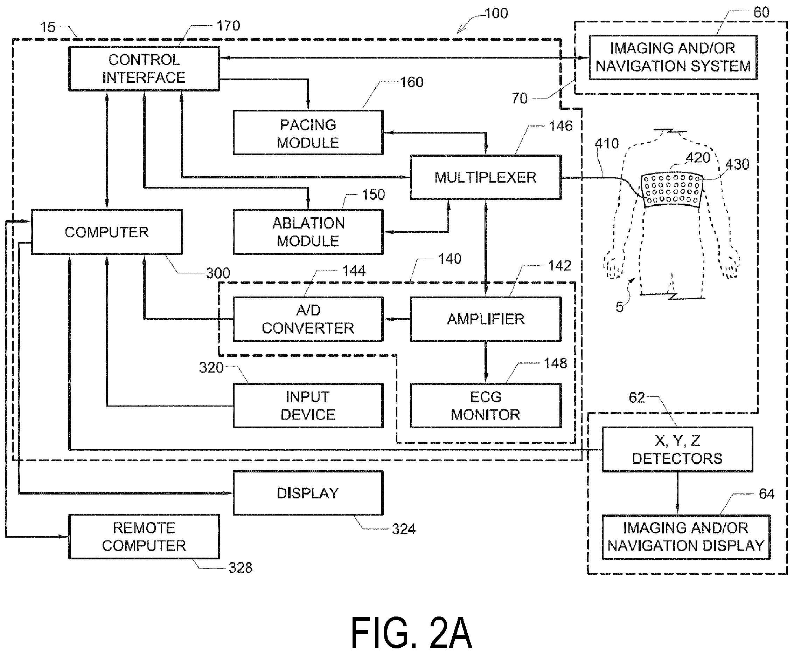

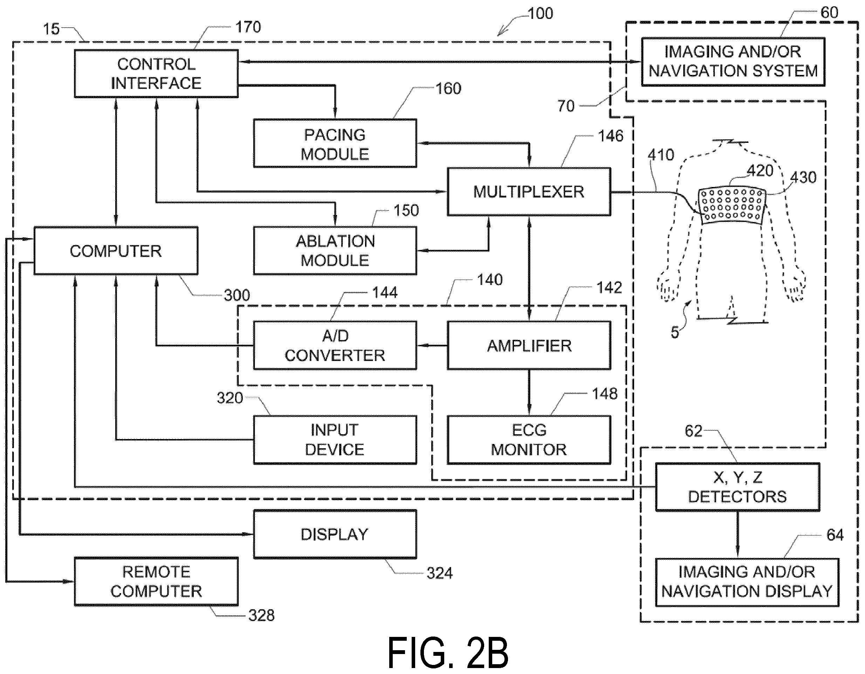

Referring now to Figs. 2A and 2B, there are shown and illustrated various aspects of extracorporeal or body surface electrode EGF systems, devices, components, and methods, which may be employed, by way of non-limiting example, to pre-screen AF patients as described above, or as diagnostic tools for determining whether a patient has AF or AT before more complicated, involved, invasive, and/or time-consuming procedures might be employed (e.g., employing an intra-cardiac basket catheter to map a patient's atrium)..

Figs. 2A and 2B illustrate two different embodiments of a combined extracorporeal body surface electrode EGF and/or cardiac electrophysiological mapping (EP), pacing and ablation system 100. System 100 shown in Figs. 2A and 2B shares many aspects and features with system 100 shown in Fig. 1B, where certain portions thereof may be interchanged, such as, by way of example, intra-cardiac pacing or ablation catheter 110 with external extracorporeal electrode vest 420, or may be removed, such as, by way of example, ablation module 150, pacing module 160, etc., depending of course on the particular application at hand. There is no need to repeat all the descriptions of the portions of systems 100 and 300 that are described above in connection with Fig. 1B, but that are shown in Figs. 2A and 2B.

In Figs. 2A and 2B there is shown a patient 5 wearing a body surface electrode vest 420 comprising a plurality of body surface electrodes 430, which are operably connected through electrical connection 410 to multiplexer 146,and thence to modules 140 and 300. Body surface electrodes 430 are configured to sense ECGs or body surface electrogram signals originating from the patient's heart. Module 140 is configured to receive such ECGs or electrogram signals through electrical connection or cable 410, and to condition such signals for further processing by computer 300. In some embodiments, electrical connection or cable 410 is replaced by a wireless connections, such as BLUETOOTH^{®} connection.

In Fig. 2A, there are shown 64 body surface electrodes 430 mounted on the anterior portion of vest 420, which in turn is worn on or attached to the thorax of patient 5. In some embodiments, another 64 body surface electrodes 430 may be mounted on the posterior surface of vest 420 (not shown in Fig. 2A). Other numbers and configurations of body surface electrodes are also contemplated, such as individual patches, multiple or interconnected patches, patches and electrodes configured to cover only certain tailored portions of a patient's torso determined, calculated, or known to provide locations for sensing optimum heart signals, and numbers of electrodes ranging, by way of non-limiting example, between 1 electrode and 3 electrodes, 4 electrodes, 8 electrodes, 12 electrodes, 16 electrodes, 24 electrodes , 36 electrodes, 48 electrodes, 64 electrodes, 72 electrodes, 96 electrodes, 128 electrodes, 256 electrodes, 512 electrodes, and 1,024 electrodes.

Some examples of current manufacturers of cardiac monitoring patches include: (a) iRhythm^{®} and their Zio XT^{®} and Zio AT^{®} Patch product offerings; (b) the Bardy Dx^{®} Carnation Ambulatory Monitor (CAM^{™}), and (c) the NUVANT^{®} Mobile Cardiac Telemetry (MCT) Monitor, which communicates wirelessly with a cellular device. See, for example: (1) U.S. Patent No. 10,123,703 entitled "Health monitoring apparatus with wireless capabilities for initiating a patient treatment with the aid of a digital computer" to Bardy et al. ("the '703 patent"); (2) U.S. Patent No. 10,299,691 entitled "Wearable monitor with arrhythmia burden evaluation" to Hughes et al. ("the '691 patent"); (3) U.S. Patent No. 10,772,522 entitled "Disposable biometric patch device" to Zadig, and (4) "Cardiac Ambulatory Monitoring: New Wireless Device Validated Against Conventional Holter Monitoring in a Case Series" to Murali et al., Front. Cardiovasc. Med., 30 November 2020 (https://doi.org/10.3389/fcvm.2020.587945) describing the SmartCardia^{®} wearable cardiac monitoring patch ("the Murali paper"). Those skilled in the art will realize that certain aspects and features disclosed and described in in the '703 patent, the '691 patent, the '522 patent, and the Murali paper can be employed in, or adapted and modified for use in, the systems, devices, components, and methods described and disclosed herein. The '703 patent, the '691 patent, the '522 patent, and the Murali paper incorporated by reference herein, each in its respective entirety. Apple iWatch^{®}, FitBit^{®}, Galaxy Watch3^{®}, and Galaxy Watch Active2^{®} are examples of watch or watch-like devices configured to acquire cardiac data from the wearer, such as ECGs, blood pressure, heart rate, etc.. Such wearable devices likewise contain certain aspects and features that can be employed in, or adapted and modified for use in, the systems, devices, components, and methods described and disclosed herein.

In the example of Fig. 2B, there are shown 32 body surface electrodes 430 mounted on the anterior portion of vest 420, which in turn is worn on or attached to the thorax of patient 5. In some embodiments, by way of non-limiting example, another 32 body surface electrodes 430 may be mounted on a posterior surface of vest 420 (not shown in Fig. 2A).

Continuing to refer to Figs. 2A and 2B, any suitable number of body surface electrodes 430 may be employed in system 100. Generally the more body surface electrodes 430 employed the better so as to improve resolution and avoid, for example, spatial aliasing of electrical signals originating from patient's heart 10 arriving at the surface of the patient's thorax. Other numbers, arrangements, configurations, and types of body surface electrodes 430 are also contemplated, however. In some embodiments, at least some body surface electrodes 430 and vest 420 are together configured to detect cardiac activation or electrical signals, and to generate electrocardiograms or body surface electrogram signals, which are then relayed by electrical conductors in cable 410 from the individual electrodes 430 to data acquisition device 140.

It is further contemplated that body surface electrodes 430 may be mounted, attached or coupled to the patient's thorax by means other than a vest, such as by patches, electrode strips, individually, or by other means known in the art. For example, electrode strips manufactured by Goltec GmbH of Cremlingen, Germany can be used. Carbon and metal body surface electrode strips are available from Goltec GmbH . Carbon electrode strips have the advantage of being radio-translucent, i.e., being transparent or substantially transparent during X-ray imaging.

Electrodes may be provided only on the anterior portion of the patient's thorax, only on the posterior portion of the patient's thorax, on side or lateral portions of the patient's thorax, or on any suitable combination of anterior, posterior and/or lateral portions of the patient's thorax.

Continuing to refer to Figs. 2A and 2B, and as mentioned above, electrodes 430 are configured to sense electrical activity originating in patient's heart 10. In addition to sensing electrodes 430, other types of devices and/or transducers, such as ground electrodes, navigation patches, position markers, or other devices may be configured to operate in conjunction with, be incorporated into, or form a portion of vest 420, electrodes 430, and/or system 10. Electrodes 430 may be reusable or disposable, unipolar or bipolar, and may be configured for use with MRT/MRI, X-Ray, and/or CAT scanning imaging systems or other types of imaging systems 60. Imaging and/or navigation system 60 may be employed used to help identify and determine the precise positions of the various electrodes 430 or position markers included in vest 430. Gels, adhesives, and liquids may be employed to improve electrical coupling of electrodes 430 with the patient's body, as is well known in the art.

Still referring to Figs. 2A and 2B, electrodes 430 configured to sense electrical activity originating in patient's heart 10 may also be individual or interconnected cardiac monitoring patches, incorporated (or not) into a wearable structure such as a vest or band. Electrodes 430 may also form portions of standard or customized 1-lead ECG monitoring leads (which typically use 1 electrode on the torso), 3-lead ECG monitoring leads (which typically use 3 electrodes on the torso), 5-lead ECG monitoring leads (which typically use 5 electrodes on the torso), and/or 12-lead ECG monitoring leads (which typically use 10 electrodes on the torso and limbs). Cardiac monitoring patches and ECG monitoring leads may have electrodes attachable to a human torso, legs or other portions of the body using adhesives suitable for that purpose, and may also comprise circuitry required to telemeter or send data therefrom via BLUETOOTH or WiFi to system 100, eliminating the need for wired connections between electrodes 430 and system 100. Such circuitry may also be configured to receive instructions, data, and programs wirelessly from system 100 or another source.

In addition to sensing electrodes 430, other types of devices and/or transducers, such as ground electrodes, navigation patches, position markers, or other devices may be configured to operate in conjunction with, be incorporated into, or form a portion of vest 420, electrodes 430, and/or system 10. Electrodes 430 may be reusable or disposable, unipolar or bipolar, and may be configured for use with MRT/MRI, X-Ray, and/or CAT scanning imaging systems or other types of imaging systems 60.

Note that in some embodiments, system 100 of Figs. 2A and 2B may not include multiplexer 146, ablation module 150, pacing module 160, imaging and/or navigation system, 60, or other modules or components shown in Figs. 2A and 2B. Among other things, the embodiments of system 100 shown in Figs. 2A and 2B are configured to detect and reconstruct cardiac activation information acquired from a patient's heart relating to cardiac rhythm disorders and/or irregularities, and is further configured to detect and discover the location of the source of such cardiac rhythm disorders and/or irregularities with enhanced precision relative to prior art techniques using body surface electrodes 430. In some embodiments, system 100 is further configured to treat the location of the source of the cardiac rhythm disorder or irregularity, for example by ablating the patient's heart at the detected source location.

The embodiment of system 100 shown in Figs. 2A and 2B comprises five main functional units: electrophysiological mapping (EP mapping unit) 140 (which is also referred to herein as data acquisition device 140), ablation module 150, pacing module 160, imaging and/or navigation system 70, and computer or computing device 300. Data acquisition, processing and control system 15 comprises data acquisition device 140, ablation module 150, pacing module 160, control interface 170 and computer or computing device 300. In one embodiment, at least one computer or computing device or system 300 is employed to control the operation of one or more of systems, modules and devices 140, 150, 160, 170 and 70. Alternatively, the respective operations of systems, modules or devices 140, 150, 160, 170 and 70 may be controlled separately by each of such systems, modules and devices, or by some combination of such systems, modules and devices.

Computer or computing device 300 may be configured to receive operator inputs from an input device 320 such as a keyboard, mouse and/or control panel. Outputs from computer 300 may be displayed on display or monitor 324 or other output devices (not shown in Figs. 2A and 2B). Computer 300 may also be operably connected to a remote computer or analytic database or server 328. At least each of components, devices, modules and systems 60, 110, 140, 146, 148, 150, 170, 300, 324 and 328 may be operably connected to other components or devices by wireless (e.g., BLUETOOTH) or wired means. Data may be transferred between components, devices, modules or systems through hardwiring, by wireless means, or by using portable memory devices such as USB memory sticks.

During body surface EP mapping or EGF analysis procedures, and as described above, body surface electrodes 430 are positioned on the thorax of patient 5, and by way of example may be mounted on a vest 420 that is configured to place individual electrodes 430 in predetermined positions on the patient's body. These predetermined electrode positions can also be provided to imaging and/or navigation system 60 and/or to computer 300 as a data file so that the spatial positions of body surface electrodes 430 are known (at least approximately), and so that EGF analysis can be carried out accordingly as described above in connection with intra-cardiac EGF analysis (e.g., as described above in connection with Figs. 1B through 10(d)).

When system 100 of Figs. 2A and 2B is operating in an EP mapping or EGF mode, body surface electrodes 430 function as detectors of electrocardiographic signals. In one embodiment, the analog signals obtained from body surface electrodes 430 are routed by multiplexer 146 to data acquisition device 140, which comprises an amplifier 142 and an A/D converter (ADC) 144. The amplified or conditioned electrogram signals may be displayed by electrocardiogram (ECG) monitor 148. The analog signals are also digitized via ADC 144 and input into computer 300 for data processing, EGF analysis and graphical display (as described above).

Note that in some embodiments of system 100 shown in Figs. 2A and 2B, and as described above, multiplexer 146 may not form a portion of system 100. In addition, in some embodiments computing device 300 may be combined or integrated with one or more of data acquisition device 140, ablation module 150, and/or pacing module 160.

### Overview

We now describe a completely different and novel approach to the problem of navigating an intra-cardiac catheter inside the human heart, and determining its position therein, which employs a purely biosignal-based navigation system that does not require the use of additional expensive and complicated navigation hardware and software systems, devices and components. In one embodiment, the biosignals employed for navigation and catheter/electrode position determination are QRS- and/or QRST-complexes originating from the lower heart chambers (ventricles). The underlying hypothesis is that the QRS-complexes and/or QRST complexes appearing in intracardiac EGMs are unique for each location in the atrium, which therefore allows location information to be extracted from them.

As employed herein, the term "navigation" may include within its scope determination of the position of a catheter, a catheter basket or splines, and/or the individual or selected ones of the electrodes mounted on or attached to the catheter basket or catheter splines inside a human heart, depending on the context. That is, some of the various embodiments of the navigation systems described and disclosed herein not only permit a distal end of a catheter to be accurately guided to one or more desired target locations inside a human heart, but also permit the 3D positions of electrodes mounted on, at or near a distal end of the catheter to be determined with a high degree of accuracy.

That such a biosignal-based navigation system actually works is demonstrated in three separate modules described and disclosed in further detail below, which relate to but one embodiment of such a system. The first module shows that a classification of an atrium, within which a QRS-complex and/or QRST-complex is recorded, is 0.779 ± 0.131 accurate using a convolutional neural network. The second module demonstrates that it is possible to detect whether EGMs are recorded with a catheter in a same position with a precision of 0.996. The third module shows that it is possible to retrieve the actual relative position of a catheter during the recording of an EGM with a precision of 8.80 ± 3.53 mm.

Under some circumstances, improved precision of the various embodiments of biosignal-based navigation systems described and disclosed herein, and that have been reduced to actual practice, may be desirable for routine clinical use. Nevertheless, here we describe the actual reduction to practice that has been achieved of several embodiments of biosignal-based navigation and catheter position determination systems, and the various devices, components and methods associated therewith. Further and more precise embodiments of biosignal-based navigation and catheter position determination systems, and the various devices, components and methods associated therewith, are also contemplated and fall within the scope of the present patent application. The navigation and position determination methods described and disclosed herein that have been reduced to actual practice clearly demonstrate that accurate navigation and positioning information can be extracted successfully from intracardiac EGMs using the novel systems and methods described herein.

AF is a condition in which the control of heart rhythm is taken away from the normal sinus node pacemaker by rapid and aberrant electrical activity occurring in different areas within the upper chambers or atria of the heart. This condition can result in rapid and irregular atrial activity and, instead of contracting, the atria quiver chaotically and arrhythmically. The current estimate for the prevalence of AF in the developed world is approximately 1.5 - 2% of the general population, and is associated with a five-fold risk of stroke, a three-fold incidence of congestive heart failure, and higher mortality. Hospitalization of patients with AF is common.

In most cases, AF is treated by pharmacological therapies. Among non-pharmacological therapies, cardiac ablation therapy is a common and well established technique. The aim of ablation therapy is to cause myocardial cell injury in a defined and localized manner along the atrial walls. To identify the targets of ablation therapy, the electrical activity of a patient's atrium needs to be understood. Therefore, a catheter with attached electrodes is placed inside one or more atria during a minimally invasive procedure. These electrodes are then used to create an intracardiac EGM by recording the electrical activity inside the atrium. This process is called mapping or electrophysiological (EP) mapping. To map the whole interior atrial surface, the catheter may be placed in multiple positions and intracardiac EGMs recorded. For the placement and navigation of the catheters, fluoroscopy imaging is typically used. The radiation exposure resulting from by the usage of fluoroscopy carries a known medical risk. In recent years non-fluoroscopic three-dimensional (3D) navigation systems have therefore been used to assist in mapping and ablation, which results in a reduction of x-ray exposure. Such 3D navigation systems permit the visualization of the catheter inside the atria.

Unfortunately, known 3D navigation systems have major drawbacks. On the one hand, they require additional hardware which is employed only for the purpose of navigation. To achieve the highest precision, such 3D navigation systems work best with a specific type of catheter, require additional body surface electrode patches on the patient's front and back, and an additional pad mounted underneath the patient table. Also, and importantly, such 3D navigation systems also generate an electromagnetic field which can result in noise and artefacts in the EGM. This is a critical factor, since EGMs are the foundation upon which treatment decisions are made on, and thus accurate and high-fidelity EGMs are important for treatment success. The foregoing negative factors regarding known 3D navigation systems illustrate the significant advantages of extracting accurate and reliable catheter navigation and positioning information from intracardiac EGMs alone. Catheter navigation and positioning information derived from intracardiac EGMs eliminates the need for costly additional hardware, and also helps minimize the introduction of noise into EGMs. To this end, signals emanating from the lower heart chambers (the ventricles), in particular QRS-complexes (and in some embodiments QRST-complexes) are utilized as described and disclosed herein, and as further described in the '249 and '605 patent applications.

As mentioned above, one embodiment of a 3D biosignal-based navigation and positioning system comprises three different modules. Going from coarse to fine, and as further described herein, these are:
- Atrium Classifier: Classifies the atrium in which an intracardiac EGM is recorded.
- Position Change Detector: Detects if two EGN recordings are taken with a catheter in the same position.
- Relative Positioning: Reconstructs catheter positions and their relative positions relative to one another.

### Heart Fundamentals

The heart is a muscular pump that is responsible for: (1) collecting oxygen-poor blood from the tissues of the body and pump this blood to the lungs to pick up oxygen and release carbon dioxide; and (2) to collect oxygen-rich blood from the lungs and pump this blood to the body's tissue.

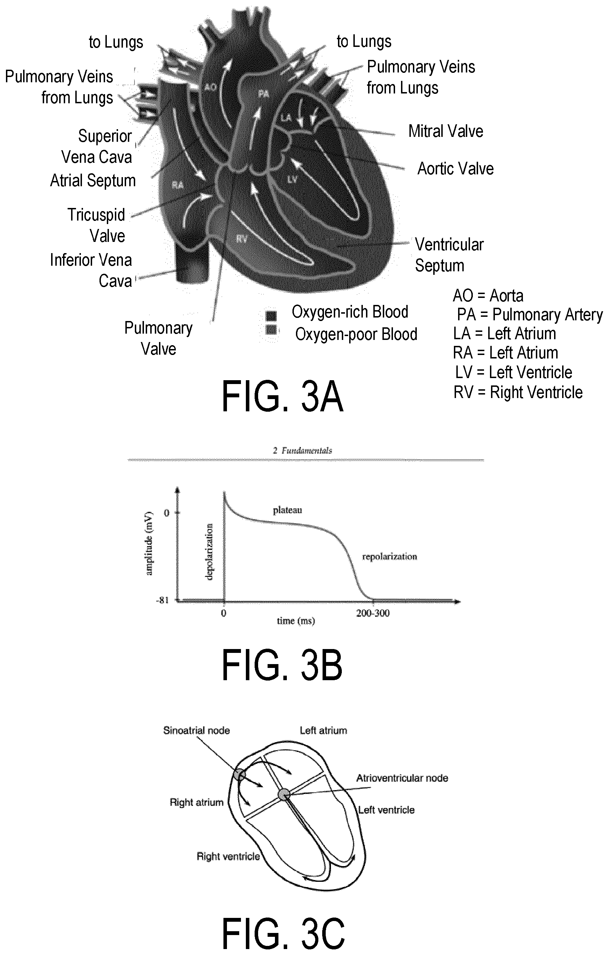

The anatomy of the human heart comprises four chambers composed of cardiac muscles or myocardium. The two upper chambers are the atria and the two lower chambers are the ventricles. The left and right halves, each consisting of an atrium and a ventricle, are each separated by a septum. These are referred to as the atrial and ventricular septa. Blood enters the right atrium (RA) of the heart through the superior vena cava. The RA contracts and pushes the blood through the tricuspid valve into the right ventricle. Then the right ventricle contracts and pushes the blood through the pulmonary valve into the pulmonary artery, which takes it to the lungs. In the lungs, the blood cells exchange carbon dioxide for oxygen. The oxygenated blood returns to the heart through the pulmonary veins and enters the left atrium (LA). Then the LA contracts and pumps the blood through the mitral valve into the left ventricle. Finally, the left ventricle contracts and pushes the blood into the aorta. The aorta branches off into several different arteries that pump the oxygenated blood to various parts of the body. See Fig. 3A, where blood flow in the human heart is visualized. In Fig. 3A, the left and right atria are located above the left and right ventricles, respectively. The atria and ventricles are separated by valves, and the left and right halves of the heart are separated by the septal wall.

### Cellular Electrical Activity

The pumping function of the heart is the result of a rhythmic cycle of contraction and relaxation of about 10¹⁰ muscle cells, a process that is controlled by a complex pattern of electrical activation. Cardiac electrophysiology is the science of elucidating, diagnosing and treating the electrical activity of the heart.

Cardiac electrophysiology is based on two general mechanisms. On the one hand, cardiac cells are excitable, i.e., they have the ability to respond actively to an electrical stimulus. On the other hand, cells are electrically connected to one other, forming a functional syncytium. Under resting conditions, cardiac cells maintain internal ionic concentrations different from those of their surroundings. Due to the electrical charge of the ions, this results in a potential difference across the cell membrane, which is called the transmembrane potential, or simply the membrane potential. More specifically, the potentials in the internal portions of cardiac cells are negative compared to their surroundings, with typical transmembrane potential values in the range of -70 to -100 mV. The actual value of the resting transmembrane potential varies between different animal species and different cell types. If an electrical stimulus is applied to a cell, the result is a change in the transmembrane potential. An excitable cell may respond to such a potential change in one of two ways. If the change is small, the conductive properties of the membrane remain unchanged, and the potential quickly returns to its resting value when the applied current is removed. However, if the applied stimulus is strong enough to raise the transmembrane potential above a certain threshold value, the response is different. In this case, the conductive properties of the cell membrane change, resulting in a rapid flux of positive ions into the cell. This causes a depolarization of the membrane, where the transmembrane potential increases from its negative resting value to a value around, or significantly above zero. After depolarization, the potential returns to its negative resting state, a process referred to as repolarization. The complete process of depolarization and repolarization is called "action potential." After depolarization, the heart muscle cells stay at their depolarized value for a significant period of time, called the plateau phase, before repolarizing. See Fig. 3B, where a typical action potential for a ventricular muscle cell is shown. In Fig. 3B, a typical human heart transmembrane voltage (in mV) is depicted over time (in ms). Depolarization is followed by a plateau, and then by a repolarization phase.

### Signal Conduction

The contraction of the heart is triggered by an electrical depolarization wave, which is passed on from cell to cell over the myocardial surface. The electrical signal in the heart starts in the sinoatrial node, which is located above the RA (see Fig. 3C). The cells in the sinoatrial node are so-called self-oscillatory cells, often referred to as pacemaker cells. This means that they spontaneously produce action potentials, and do not rely on any external stimulus. The electrical activation of the sinoatrial node stimulates neighbouring atrial muscle cells. The muscle cells are connected by so-called gap junctions, which are large proteins that form channels between adjacent muscle cells. These channels allow a flux of electrical current in the form of ions, and therefore provide a direct electrical connection between the internals of neighbouring cells. As soon as one cell is depolarized this coupling will affect the potential in neighboring cells, and may raise the transmembrane potential above the threshold value. Stimulating a small region of the atria hence results in a propagating wavefront of depolarization, which activates the complete atria and causes them to contract.

The atria are separated from the ventricles by an electrically nonconductive layer, and as a result the depolarization wavefront does not propagate directly into the ventricles. Instead, the only place where the electrical signal can be transmitted to the ventricles is through the atrioventricular node. See Fig. 3C, which is a schematic view of the pathways of electrical conduction in the heart. Activation starts in the sinoatrial node, and then activates the atria, after which the electrical signal is conducted through the atrioventricular node to activate the ventricles. Conduction of the electrical signal through the atrioventricular node is quite slow, which results in a small time delay between the activation of the atria and the ventricles. The effect of this delay is that the atria contract while the ventricles are still relaxed, which improves filling of the ventricles and the pumping function of the heart. From the atrioventricular node, the electrical signal enters the atrioventricular bundle. From there, the muscle cells inside the ventricle are stimulated. Like the atrial cells, the ventricular muscle cells are also connected by gap junctions. The stimulus therefore causes wavefronts of depolarization, which eventually activates the complete mass of the ventricles, and triggers the contraction of the cells.

### Analyzing the Electrical Activity of the Heart

To understand and analyze the electrical activity of the heart, ECGs and intracardial EGMs can both be used. While for an ECG the electrodes used to record the signals are placed on the body surface, for an EGM they are placed inside the heart, i.e., within the atria. While an ECG can generally only record the mean electrical heart vector resulting from all the electrical activity in the heart, an intracardiac EGM permits analysis of the electrical activity in the heart with a much more local field of view.

Since the depolarization wave has a positive potential in the front and a negative potential in the back, a wavefront traveling towards an electrode results in a positive voltage. A wavefront traveling away from an electrode results in a negative voltage. The closer the wavefront to the electrode, the higher the absolute measured voltage . A more detailed description of this phenomenon is provided below.

### Electrocardiograms (EGMs)

An electrocardiogram (ECG) is a recording of electrical potential differences on the body surface that result from the electrical activity in the heart. The potential differences are called leads. One standard ECG recording method employs 12 leads. Since it is only possible to measure electrical potentials at a point with an electrode, only potential differences can be measured. Potential differences measured by the 12 leads of a standard ECG are defined below in Table 1. For each lead the potential difference is defined by *θ_{Exploring} - θ_{Zero}.* If there are multiple *Zero* electrodes, they will construct a zero electrode together by averaging. The six leads V1-V6 were constructed by placing six electrodes on the front of the chest.

**Table 1: The leads of a 12-lead ECG. The notations LL, LA, and RA are used for the left leg, left arm, and right arm electrodes, respectively. The six chest electrodes are denoted 1-6.**

| Lead | Exploring | Zero |
|---|---|---|
| I | LA | RA |
| II | LL | RA |
| III | LL | LA |
| aVR | RA | LA and LL |
| aVL | LA | RA and LL |
| aVF | LL | RA and LL |
| V1-V6 | 1-6 | RA, LA, and LL |

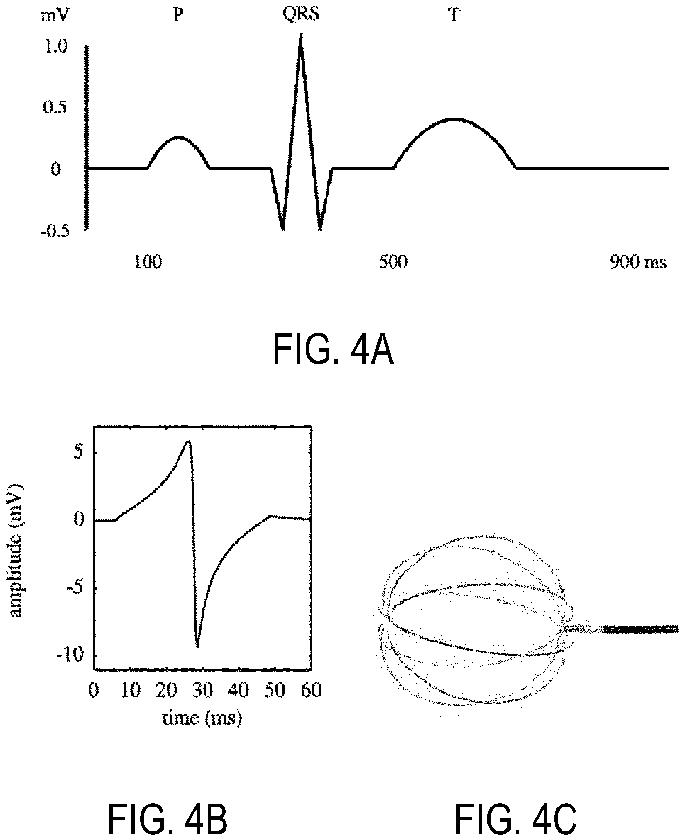

A schematic view of a typical ECG, i.e., a recording from one of the leads, is shown in Fig. 4A, where time along the x-axis is in ms) and potential difference along the y-axis is in mV. The characteristics of the heart signal are noted on top. The first signal is the P-wave, followed by the QRS-complex and then by the T-wave. The straight line segments in the ECG, where the recorded potential difference is zero, correspond to intervals in the heart cycle when there are no sources of electrical current in the heart. The five deflections in the ECG are the result of current sources arising in the heart during the electrical activation of the tissue. The five deflections are noted as P, Q, R, S and T. The first signal is the P-wave, which is the result of the depolarization of the atria. The next three signals form a combined signal called the QRS-complex, corresponding to depolarization of the ventricles. The large amplitude of this signal compared to the P-wave is a result of the larger mass of the ventricular muscles. The last signal of the normal ECG is called the T-wave, which is the result of the repolarization of the ventricles.

### Intracardiac Electrograms

As opposed to an ECG, an intracardiac EGM records the potential differences inside the heart, and in particular those occurring inside the atrium. The recording electrodes are attached to a basket or other type of EP mapping catheter. During an EP mapping procedure, a physician inserts the catheter into the RA. To enter the LA, the septum is punctured.

An example of an EGM can be seen in Fig. 4B, which shows a unipolar EGM. In clinical practice, EGMs are measured between one point on a cardiac surface and a reference electrode, which is usually a patch electrode located at the body surface. As mentioned above, electrodes for an intracardiac EGM are attached to a catheter. In one embodiment, a catheter configured to cover large portions of an atrial wall is a so-called basket catheter, and has 64 electrodes arranged on 8 splines, each spline having 8 electrodes mounted thereon or attached thereto. Splines are generally named alphabetically, and electrodes are enumerated (e.g., the third electrode from the tip of a basket on a second spline is referred to as electrode B3). Most basket catheters are made of Nitinol, and are elastically deformable. One embodiment of such a basket catheter is illustrated in Fig. 4C, where a FIRMap*^{™}* basket catheter with eight splines, each spline having eight electrodes, is shown.

### Artificial Neural Networks and Feed Forward Networks

The goal of a feedforward network is to approximate some function *f**. For example, for a classifier, *y = f**(*x*) maps an input *x* to a category *y.* A feedforward network defines a mapping *y = f*(*x*; *θ*) and learns the value of the parameters *θ* that result in the best function approximation. During neural network training, for each example x from the training data, the parameters *θ* are adjusted, so that the output of the network of the given example *x* is close to the expected label *y.* This difference between the output and the expected label y is called loss. The adjustment of *θ* is done using a gradient-learning approach.

A feedforward neural network consists of multiple layers, with a layer being defined as multiple neurons. All neurons of one layer are connected with all neurons of the next layer. Those neuron to neuron connections are weighted. Each neuron also has a bias, which is just a scalar value. The output of a neuron is the sum of the weighted inputs ${\sum }_{i}^{n} {w}_{i} {x}_{i}$ plus the bias *b*. This output is then fed into a non-linear activation function *a*, so that the network can also learn non-linear functions. The overall function of a single neuron is defined as: $a \left({\sum }_{i}^{n} {w}_{i} {x}_{i} + b\right) ,$ with n being the number of neurons in the previous layer.

### Convolutional Neural Networks

Feed forward networks have the major drawback that each neuron has its own connection, *i.e.,* weight to each neuron in the next layer. Using multiple layers with multiple neurons lets the number of weights explode very quickly.

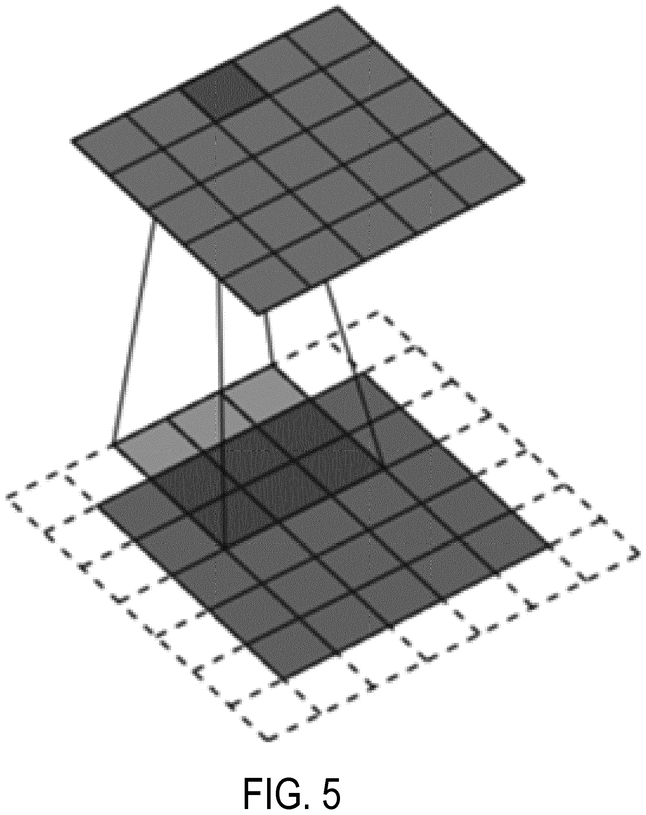

Convolutional neural networks make use of weight sharing. Convolutional layers consist of a set of kernels (also called filters) that are convolved with the input. A convolution can be seen as a dot product between the kernel and the input. All values of the input are multiplied by their corresponding value (also called weighting) in the kernel. Then all values are summed up resulting in a scalar output. One example of a convolution operation is visualized in Fig. 5.Stacking multiple convolutional layers allows a network to learn more and higher level features. For example, in image processing the first convolution layers learn edges and corners, and then later layers learn full objects. By way of example, in Fig. 5 a 3 × 3 kernel is convolved over a 5 × 5 input (blue) zero padding and unit strides, resulting in the output (green).

### Autoencoders

An autoencoder is a neural network that is trained to output a given input. Internally, it has a hidden layer h that describes a code used to represent the input. The network may be viewed as comprising two parts: an encoder function *h = f(x),* and a decoder that produces a reconstruction r *= g(h)* . Undercomplete autoencoders constrain *h* to have a smaller dimension than *x.* Learning an undercomplete representation forces the autoencoder to capture the most salient features of the training data. The learning process may be described simply as minimizing a loss function *L (x, g(f(x))),* where *L* is a loss function penalizing *g(f(x))* for being dissimilar from *x.* Regularized autoencoders provide the ability to train an autoencoder which fulfills other properties besides reconstructing the input.

Autoencoders exploit the idea that data concentrates around a low-dimensional manifold or a small set of such manifolds. Autoencoders are configured to learn the structure of a manifold. An important characterization of a manifold is the set of its tangent planes. At a point *x* on a *d*-dimensional manifold, the tangent plane is given by *d* basis vectors that span the local directions of variation allowed on the manifold. These local directions specify how one can change *x* infinitesimally while staying on the manifold.

All autoencoder training procedures involve a compromise between two forces:
1. Learning a representation *h* of a training example *x,* so that *x* can be approximately recovered from *h* through a decoder.
2. Satisfying the latent constraint or regularization penalty.
The underlying idea is that the two forces together force the hidden representation to capture information about the structure of the input data. An important principle is that an autoencoder can afford to represent only the variations that are needed to reconstruct training examples. If the input data concentrates near a low-dimensional manifold, this yields representations that implicitly capture a local coordinate system for the manifold: only the variations tangent to the manifold around *x* need to correspond to changes in *h = f*(*x*). Therefore the encoder learns a mapping from the input space *x* to a representation space, a mapping that is only sensitive to changes along the manifold directions, but is insensitive to changes orthogonal to the manifold .

### Related Work and 3D Navigation Systems

In the past, fluoroscopy imaging was the primary tool used to navigate catheters into and through the atria . Since fluoroscopy employs x-rays, they expose to physicians to medical risk. In recent years, new technologies have entered the market with the goal of reducing the use of fluoroscopy for catheter navigation. Systems configured to visualize and help navigate catheters inside the atria are called 3D navigation systems. There are three major types of technologies these kinds of systems employ, which are now described below.

### Impedance-Based Technology

Impedance means resistance to electrical current. Several patches are placed on the patient's body. These patches are in pairs. A single frequency current is sent from patch to patch and a baseline impedance is measured. When a catheter is inserted between the patches, this creates additional impedance. The change in impedance is measured, which allows the position or location of the catheter to be determined.

### Magnetic Technology

A magnetic field is generated by a pad which is located beneath the operating table that codes the mapping space around the patient's chest with temporal and spatial distinguishing characteristics. The field contains all information necessary to resolve the six degrees of freedom of the catheter (location and orientation). To this end, a catheter is equipped with a sensor which is capable of measuring the magnetic field and then a processing unit that encodes this measurement into a position .

### Current-based Technology

A catheter is placed inside the patient's body and emits a current at a unique frequency. Patches on the body surface collect the current and measure its strength at each patch. This information is then used to calculate a current-ratio. The current-ratio is the average of the current that all patches on the body surface receive from an electrode. This value is unique to a location. Some 3D navigation systems combine different technologies, such as the CARTO 3^{™} system, which uses magnetic and current-based technology. All three of the foregoing navigation technologies require additional hardware that is only used for navigation and positioning, which increases medical costs. Some navigation systems apply a current inside the patient which is configured to disturb the recording of EGMs. Here, the methods we have developed only use the heart signal itself for navigation and positioning, do not require any additional hardware, and do not result in any disturbances to EGMs.

In the general case, 3D navigation systems require a precision of 3 mm or less. The precision of two commonly used navigation systems, the CARTO 3 System and the EnSite system, were assessed resulting in point localization performances of 0.46 ± 0.17 mm and 0.79 ± 0.83 mm, respectively.

### Biosignal Based Intracardiac Navigation System Atrium Classification

One embodiment of the atrium classifier described and disclosed herein uses a neural network for atrium classification. In recent years, there have been several works describing the application of artificial neural networks to intracardiac EGM analysis. However, we are unaware of any work where intracardiac EGMs are used to extract position or navigation information. Most works focus on classifying or detecting abnormalities such as atrial fibrillation patterns , ventricular abnormal potentials, or differentiating between multiple arrhythmias.

### Position Change Detector

In one embodiment, a position change detector uses an approach from the field of computer vision, which is called shape matching . A common approach is to use point descriptors from two shapes, measure their similarity, and from there resolve corresponding points. In computer vision, for example, point descriptors can be associated with brightness or color In one embodiment of a position change detector, a pointwise descriptor is a recorded EGM signal.

### Relative Positioning

A relative positioning algorithm is also a neural network, and builds on the idea of manifold learning. Manifold learning describes the process of learning a function which maps a high dimensional input space to a low dimensional representation space which is only sensitive to changes along the manifold directions, but which is insensitive to changes orthogonal to the manifold.

The first unsupervised attempts of learning a lower dimensional manifold we employed were non-parametric methods based on nearest-neighbor graphs. Each node in the graph was a training example and the edges connect near neighbors to one another. Unfortunately, if the manifold is not smooth, a large amount of training data is required to cover the whole manifold. Autoencoders were found to work best since they were discovered to outperform other manifold learning algorithms.

For relative positioning, a representation space can be constrained by pairwise distances between electrodes in a catheter, and can be employed to approach the problem of localizing the position of user equipment similar to that employed in wireless systems. They can be used to train a Siamese neural network and constrain the representation space using pairwise distances. However, this is the first known work which applies such a similar algorithm using EGMs to extract position information from a heart signal.

### Dataset

For quantitative and qualitative performance assessment of the methods described and disclosed herein, a proprietary dataset was used. The dataset consisted of 432 recordings from 17 patients. Each recording contained a standard 12 lead body surface ECG and an intracardiac EGM. The body surface electrodes were not moved during the procedure. When acquiring intracardiac EGMs, a deformable 64-electrode basket catheter (FIRMap^{™}, Abbott, Abbott Park, IL) was employed. The diameter of the basket catheter was either 50 mm or 60 mm. As described above for the treatment of AF, the whole atrium in each patient was mapped (i.e., the basket catheter with its attached electrodes was placed in multiple positions within the same atrium).

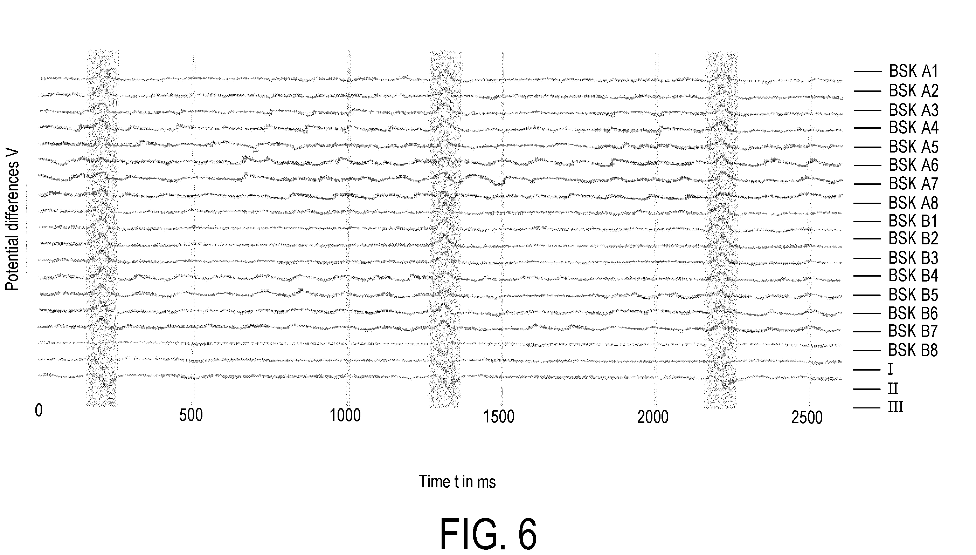

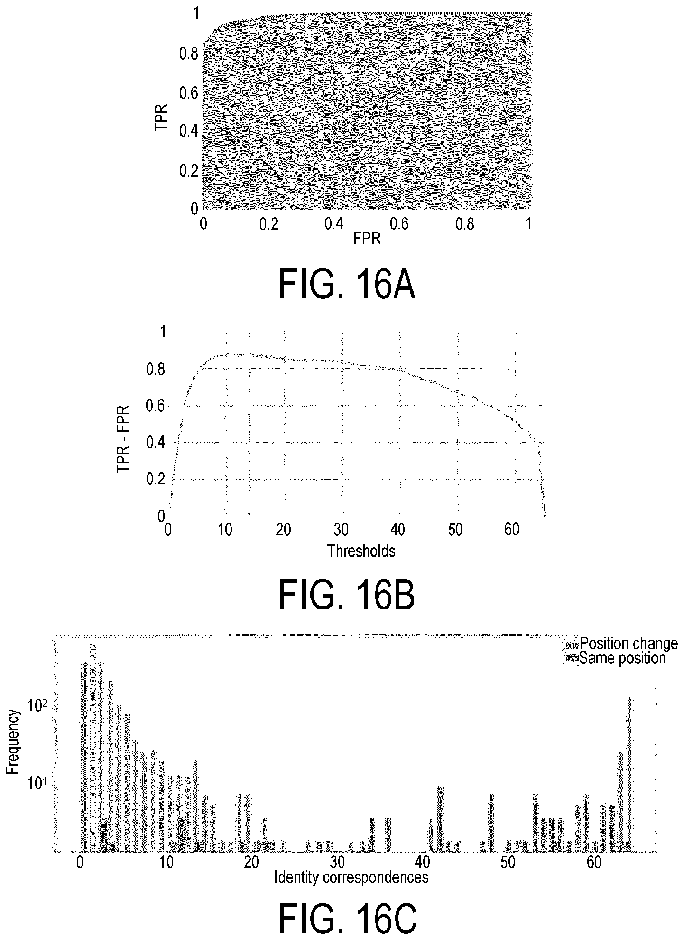

At each position, at least three recordings, each ~60 seconds in length, were captured. See Fig. 6, where there are shown the results of simultaneous recording of intracardiac EGMs and body surface ECGs. In Fig. 6, 16 channels from two splines of a basket catheter are shown (BSK A1 - BSK A8, BSK B1 - B8), and three of the twelve leads from a body surface ECG are visualized. Highlighted in red are the QRS-complexes. The time span of the recordings is 2.6 seconds.

A descriptive analysis, and the split between training/validation and test results, are shown together in Table 2 below. The dataset split is the same for all introduced methods.

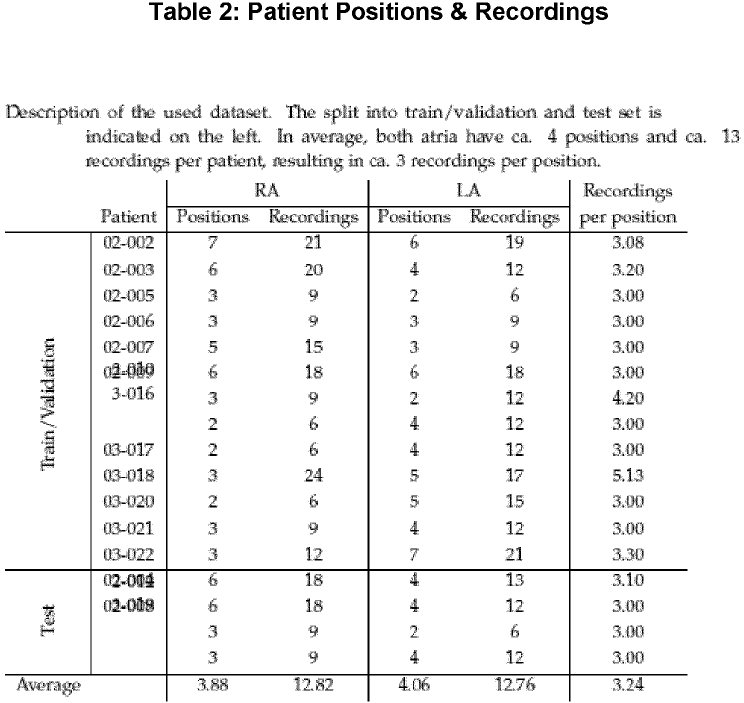

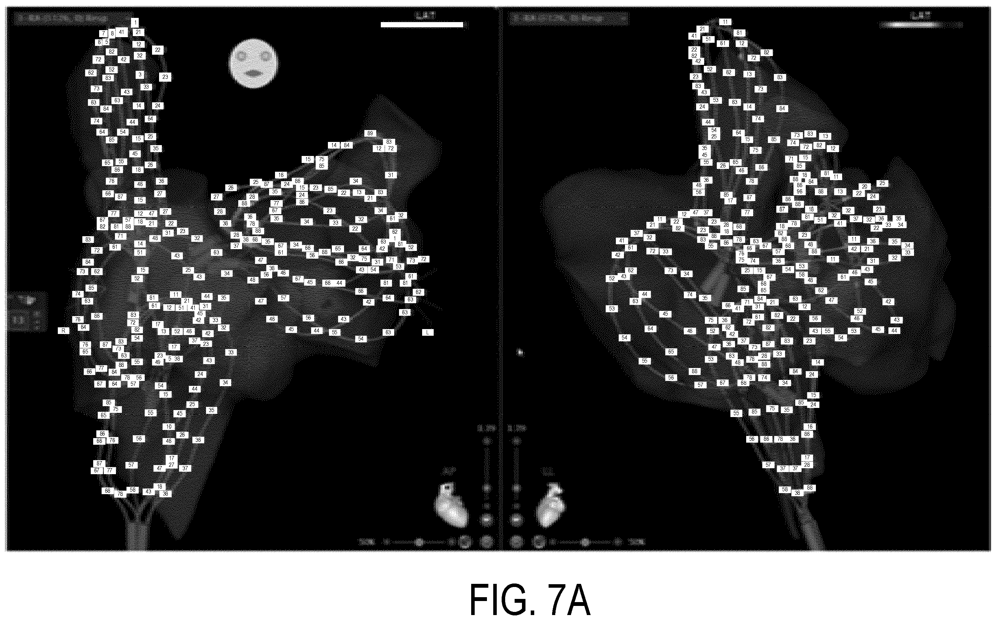

During all procedures, a 3D navigation system (i.e., CARTO 3) was used to localize and navigate a catheter inside the atria. For each position, two screenshots from an orthogonal view were taken from which the 3D locations of each electrode could be reconstructed (see Fig. 7A). Those reconstructions served as ground truths to compare the estimates of the presented methods against one another. Fig. 7A shows one example of a screenshot from the real time CARTO 3navigation system: one of a left anterior-posterior view, and another of a right left-lateral view. The two views are orthogonal to each other. Three positions in the RA are shown, as are two positions in the LA. There is always only one catheter at a time. The image shows a superimposition of multiple time points, where the catheter was in different positions. In Fig. 7A, electrodes are enumerated by a first number corresponding to a spline, and a second number corresponding to an electrode on the spline (e.g., the third electrode on the second spline is denoted 23).

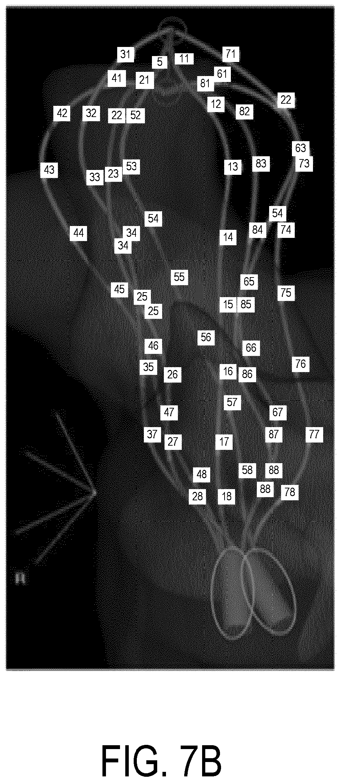

Fig. 7B illustrates some limitations associated with the navigation system employed to provide the results shown in Fig. 7A. A single eight spline basket catheter is visualized as two basket catheters with each having four splines. The red circles and the blue circles indicate positions which are in reality the same, but that wind up being visualized in different locations.

Although the CARTO 3 navigation system employed in connection with Figs. 7A and 7B has a claimed precision of 0.46 + 0.17 mm, such precision only applies to a specific catheter type where CARTO 3 can make use of its current-based and magnetic technology, as further described below. The catheter we used during data acquisition, however, did not support or work in conjunction with magnetic technology, and thus the navigation system can only operate in a current-based mode. This likely reduced the accuracy of the employed navigation system. Unfortunately, it is not feasible in this work to quantify the actual resulting precision or error.

Additionally, and as shown in Fig. 7B, the CARTO 3 system visualized the employed basket catheter with eight splines as two catheters, with each catheter having four splines. Moreover, the tips and poles of the two visualized basket catheters do not align (shown in Fig. 7B as red and blue circles), even though there is actually only a single basket catheter, and they are in the same position.

### Methods

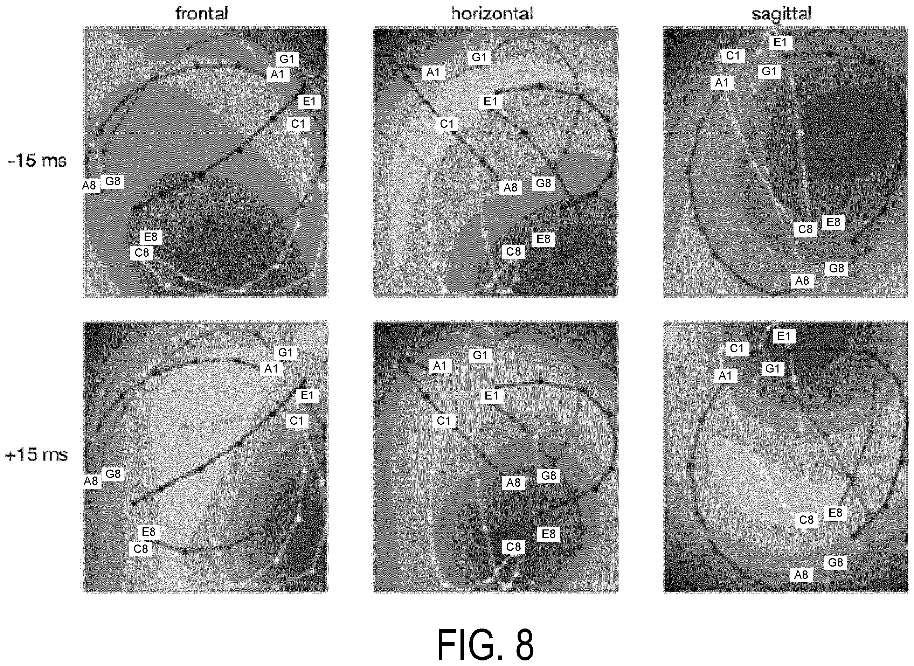

The QRS-complex is the result of the depolarization of the ventricles. Our discovery is that the QRS-complex in an intracardiac EGM contains position information regarding electrode positions and locations. We have discovered that an electrode placed in the atria observes the ventricular depolarization from a location-dependent and unique field of view, which then results in a unique morphology of the QRS-complex. It is important to note, however, that this uniqueness appears only to hold for the full duration of the QRS-complex (e.g., 80 - 100 ms), and not for a single time point within this QRS-complex, which is illustrated in Fig. 8. While there are equal potential differences at one time point, there is no equal potential difference for two coordinates at different time points. Fig. 8 shows the contours of potential differences at two time points. In Fig. 8, the upper row of subplots represents 15 ms before the R-peak of a QRS-complex, and the lower row of subplots represents 15 ms after the R-peak of a QRS-complex. The respective columns shown in Fig. 8 correspond to frontal, horizontal and sagittal views, respectively. Each line in each subplot represents a catheter spline, and the markers on the lines represent the electrodes. The same background color in each subplot indicates a same potential difference range.

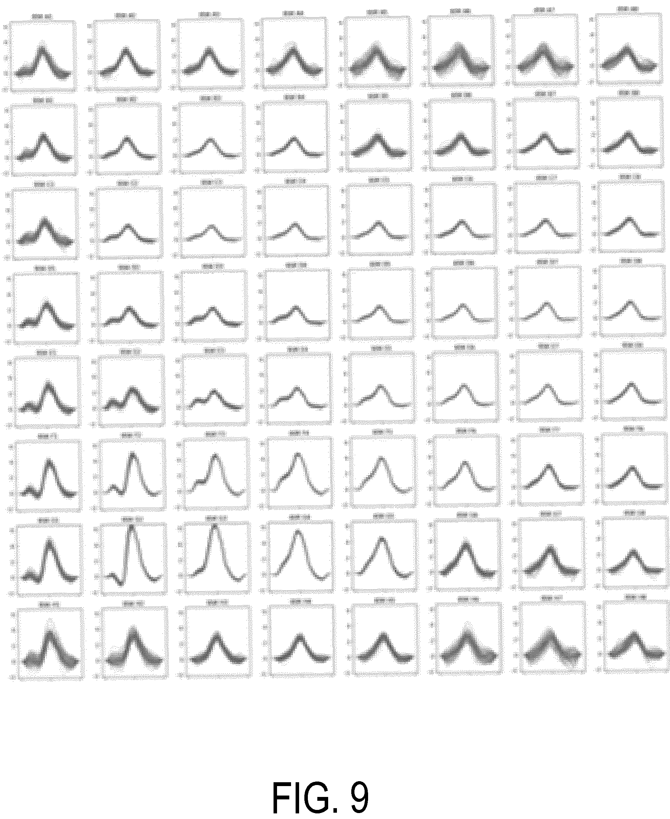

Fig. 9 shows a series of extracted QRS-complexes for ~60 second EGM recordings. Each subplot shows all the QRS-complexes recorded in a given or certain electrode. The visualizations shown in Fig. 9 support the hypothesis that a QRS-complex has a unique shape that is location dependent. In Fig. 9, all N QRS-complexes were recorded by their respective electrodes over a time period of 60 seconds. Each subplot shows all N QRS-complexes extracted within those 60 seconds at the respective electrode. While there are some small deviations within some subplots/electrodes (e.g., BSK A6), many others have almost no deviations (e.g., BSK D7).

The observed signal during the depolarization of the heart can be described as a function: $V \left(x, t\right) ∈ ℝ ,$ with t being a time point during the QRS-complex and x being the 3D location inside the atrium, and thus $x ∈ {ℝ}^{3}$. Since the depolarization of the ventricles and therefore the full QRS-complex is not only a single time point but lasts 80 - 100 ms, this equation can be formulated as: $V \left(x, t\right) ∈ {ℝ}^{T}$ with $t ∈ {ℕ}^{T}$ and T being the duration of the QRS-complex. Considering C locations at once, the equation can then be formulated as: $V \left(X, t\right) ∈ {ℝ}^{C \times T} ,$ with $X ∈ {ℝ}^{C \times 3} , C ∈ {ℕ}^{+}$. In the discrete case, this function can be encoded as the matrix: ${V}_{X} ∈ {ℝ}^{C \times T} ,$ which describes the potential differences over a time span T in C locations.

As stated above, an intracardiac EGM is usually multiple seconds or minutes in duration. Within that time, the heart beats N times (usually 60 - 120 times per minute). This results in N QRS-complexes, and since a QRS-complex is also a function of space, it also results in *N* locations X. One of those *N* locations may be encoded by **X**ᵢ with $i ∈ {ℕ}^{N}$. The catheter is not moved during the EGM recording, and thus **X**ᵢ should be the same. However, since the heart is a moving organ there are slight movements. Thus the average location of a catheter during a recording ${X}_{avg} ∈ {ℝ}^{C \times 3}$ is defined by: ${X}_{avg} = \frac{1}{N} {\sum }_{i}^{N} {X}_{i} .$ Therefore, the average QRS-complex ${V}_{{X}_{avg}} ∈ {ℝ}^{C \times T}$ is also defined by: ${V}_{{X}_{avg}} = \frac{1}{N} {\sum }_{i}^{N} {V}_{{X}_{i}} .$

### Preprocessing and Isolation of the Ventricular Signal

The signal **V** within an ECG, but also within an intracardiac EGM, is a superposition of several independent components. These components may be defined as:
- **V***ₐₜᵣᵢₐₗ*: voltage differences originating from the wavefronts in the atria.
- **V***_{ventricular}:* voltage differences originating from the wavefronts in the ventricles.
- **V***_{baseline-wander}:* a low-frequency high-amplitude artefact.
- **V***ₘₐᵢₙₛ:* sinusoid component of narrow frequency bands originating from nearby electronic devices.

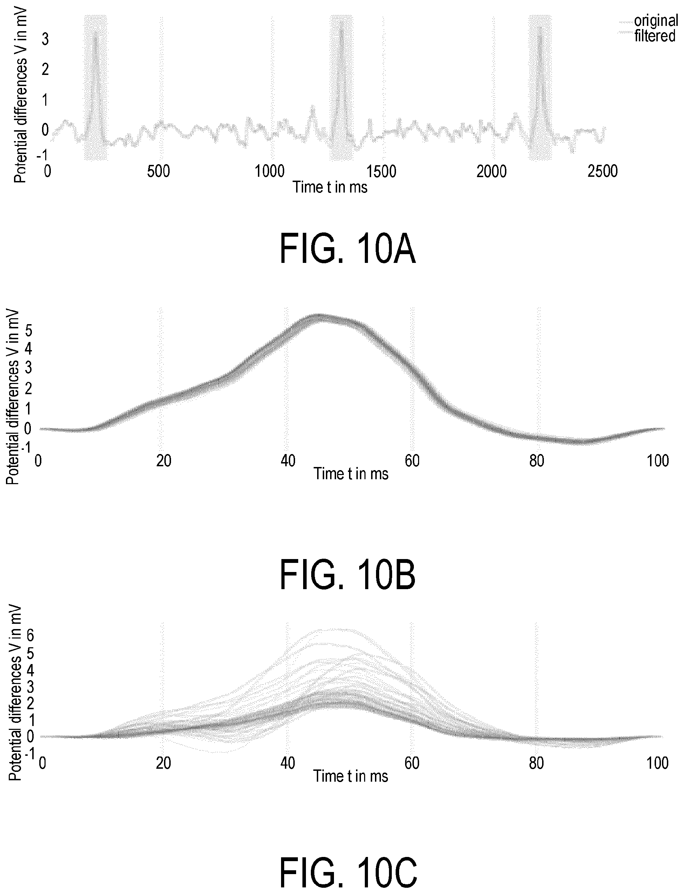

Most embodiments of the methods described herein employ QRS-complexes of intracardiac EGMs, although QRST complexes can also find application in such methods. Since the QRS-complex is part of **V***_{ventricular},* the other components must be removed. One embodiment and example of an EGM signal before and after isolation is shown in Fig. 10A, where the original unfiltered signal is shown in blue, and the filtered signal is shown in red. The highlighted areas are the QRS-complexes, and the time span is 2.5 seconds.

### Extraction of QRS-Complexes

Note that in addition to the description and disclosure provided herein, extraction and isolation of QRS and/or QRST complexes is further described and disclosed in the '249 and '605 patent applications.

Atrial activity is less pronounced body surface ECGs than in intracardiac EGMs, which leads to less distortions of the actual QRS-complex in body surface ECGs. Since EGMs and ECGs are recorded in parallel (e.g., see Fig. 6), QRS-complexes occur synchronously in both modalities. Therefore, the time window for the QRS-complex can be determined using a body surface ECG. To this end, the same preprocessing steps described above may applied to an ECG. In a resting patient, QRS-complexes are usually very consistent, which permits so-called templates to be built from them, and which represent a mean QRS-complex per electrode. The synchronization point to align QRS-complexes is the strongest slope which corresponds to an R-peak. After determination of the time points of the R-peak, QRS-complex windows can be extracted from an EGM.

Afterwards, a clustering algorithm (DBSCAN ) is applied to identify clusters of QRS-complexes. Here, only the most dominant cluster may be considered. Examples of extracted QRS-complexes are shown in Figs. 9, 10B and 10C. Fig. 10B shows N QRS-complexes extracted from a single location x. Each line represents one QRS-complex. The extracted QRS-complexes correspond to electrode BSK G4 in Fig. 9. Fig. 10C shows N averaged QRS-complexes from the 64 electrodes of the catheter. Each line represents an average QRS-complex for one electrode. Fig. 10C illustrates the uniqueness of the QRS-complex, since no one QRS-complex has the exact same morphology as the others.

### Atrium Classifier

When building a 3D navigation system, it is important to know in which atrium the basket catheter is to be placed. Therefore, the first attempt of extracting position information from a QRS-complex is by classifying in which atrium (left or right) a basket catheter is to be placed while an EGM is recorded.

Heart physiology is similar across patients (see reference [3] in the bibliography). The left ventricle usually has a stronger depolarization than the right ventricle, since there are more cardiac cells in the left ventricle than in the right ventricle. One might think that this boils down to amplitude comparison of intracardiac EGMs. Unfortunately, this is not true since the observed signal is a combination of all ventricular activation.

However, the QRS-complex observed in the right atrium is systematically different from that of left atrium. Therefore, a 3D convolutional neural network can be trained to classify the atrium in which a given QRS-complex of an EGM has been recorded. For the loss of binary cross entropy: $L \left(y, \hat{y}\right) = - {\sum }_{i = 1}^{n} {y}_{i} ⋅ log {\hat{y}}_{i}$ with *y* being the ground truth, and *ŷ* being the prediction of the atrium one-hot encoded.

Since an EGM recording has a duration of multiple seconds/minutes in the same position (i.e., within the same atrium), and thus records N QRS-complexes **V**_{**X***ᵢ*}, they should all have the same morphology. To increase robustness against noise, an average QRS-complex per electrode over a full recording **V**_{**X***_{avg}*} is advantageously used as an input to the network. The average QRS-complex per electrode **V**_{**X***_{avg}*} is reshaped to ${ℝ}^{\sqrt{C} \times \sqrt{C} \times T}$, so that the 3D convolutional network can extract maximum information by incorporating spatial relationships in the convolutional filters across channels. The output is the estimated atrium in which the QRS-complex was recorded.

### Position Change Detector

During a procedure, and in one embodiment, the catheter is placed in multiple positions in the heart of a patient. A position is defined by the 3D locations of all the electrodes of the catheter. At each position, at least three recordings, each ~60 seconds in duration, are recorded. Since the heart is beating, it may occur that the catheter moves unintentionally during the procedure, without the physician noticing it. This can have an impact on a procedure's outcome because drivers of AF can be missed, thus preventing success in subsequent ablation therapy. A position change detector can be of great help because it can detect basket catheter movements and make the operating physician aware of such movements, if they occur.

Given two basket catheter positions denoted by $X , Y ∈ {ℝ}^{C \times 3}$, which are in the same position if **X** = **Y.** The discrete representation of a QRS-complex at the locations **X** and **Y** are **V_{X}** and **V_{Y}**, respectively. If V_{X} = **V_{Y}** holds, it follows that **X** = **Y.** This means that a baskets catheter is in the same position if it observes the same QRS-complex, and vice versa. This allows a classifier to be built that discriminates whether recordings are done in the same position.

Since the observed signal is not only the ventricular depolarization but also includes other components such as noise (which may not be fully removed from the signal during the preprocessing), only comparing signals is not sufficient. In addition, the heart is a moving organ, and thus a catheter positioned inside an atrium may move slightly as well.

Position change detection can be formulated as a correspondence problem, which is known in the field of computer vision. An algorithm disclosed in reference [34] of the Bibliography hereof may therefore be employed and modified for use as a position change detection. Given two three-dimensional objects **X** and **Y,** the task is to find a meaningful correspondence *φ*: **X** → **Y.** The correspondence mapping *φ* should fulfill the following properties:
1. Bijective.
2. Nearby points on **X** should be mapped to nearby points on **Y** (and vice versa).
3. Similar points should be put into correspondence.

The first point is a simplification, because if there is no overlap between two positions the resulting correspondence *φ* would not be meaningful. However, the hypothesis is that the discrimination between a reasonable and unreasonable correspondence is possible, which still allows discrimination between same position and different position recordings.

The bijective matching of correspondences *φ* can be represented by a permutation matrix Π ∈ {0,1)^{C×C} satisfying **Π^{T}1 = Π1 = 1** with 1 being a column vector of ones and C being the number of points in the matching. The space of C × C permutation matrices is denoted *by P_{C}.* The correspondence problem may be phrased as an energy minimization problem: $Π * = arg {min}_{Π ∈ {P}_{C}} E \left(Π\right) ,$ with *E*(Π) as the weighted aggregate of two terms: $E \left(Π\right) = αg \left(Π\right) + h \left(Π\right) .$

The first term g(Π) is a data term trying to align a set of pointwise descriptors encoding the similarity between points. The second term h(Π) is a regularization term promoting the continuity of the correspondence by aligning a set of pairwise descriptors encoding global/local relations between pairs of points . The parameter α weights those two terms against each other.

### Pointwise Descriptors

Similarity of points can be measured with the help of pointwise descriptors. For the purpose of position change detection, pointwise descriptors are the potential differences of all electrodes corresponding to positions **X** and **Y,** i.e. ${V}_{X} ∈ {ℝ}^{C \times T}$ and ${V}_{Y} ∈ {ℝ}^{C \times T}$ respectively. This gives rise to the optimization problem: ${argmin}_{Π ∈ {P}_{C}} {\left‖{ΠV}_{X}-{V}_{Y}\right‖}^{2} = {argmax}_{Π ∈ {P}_{C}} \left〈Π,{V}_{Y}{V}_{X}^{⊤}\right〉 ,$ which is optimizing for the optimal permutation matrix **Π,** to minimize the potential differences between **V_{X}** and **V_{Y}.**

### Pairwise Descriptors

To model the second property of *φ*, pairwise descriptors of the form ${D}_{X} , {D}_{Y} ∈ {ℝ}^{C \times C}$ are used. These methods aim for solving optimization problems of the form: $\begin{matrix}Π * = {argmin}_{Π ∈ {P}_{C}} {\left‖{ΠD}_{X}-{D}_{Y}Π\right‖}^{2} \\ = arg {max}_{Π ∈ {P}_{C}} \left〈Π,{D}_{Y}{ΠD}_{X}\right〉 ,\end{matrix}$ which is known as the quadratic assignment problem.

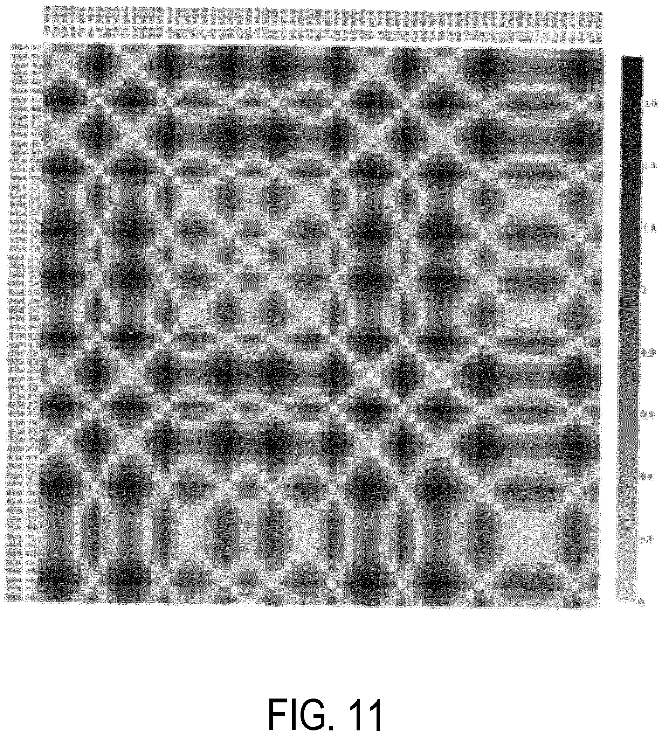

A common choice for pairwise descriptors is geodesic distances. For the purpose of position change detection, pairwise descriptors are the euclidean distances between all C electrodes, where an undeformed spherical basket catheter is assumed. Since this assumption holds for both pairwise descriptors **D_{X}** and **D_{Y}**, therefore **D_{X}** = **D_{Y}**. This assumption is a simplification because the basket catheter may be deformed. However, to fulfill **X** = **Y** this deformation is applied to both descriptors, which is simplified to no deformation applied to either of them. This distance matrix for an undeformed spherical basket is shown in Fig. 11, where a pairwise distance matrix of an undeformed spherical basket catheter with unit radius is shown. Each entry in the matrix represents the euclidean distance between two corresponding electrodes. Since the euclidean distance is symmetric, the distance matrix is symmetric.

### Optimization

Recalling the equation: $Π * = arg {min}_{Π ∈ {P}_{C}} E \left(Π\right) ,$ and inserting the pairwise and pointwise descriptors therein, gives rise to the optimization problem: $Π * \begin{matrix}= arg {min}_{Π ∈ {P}_{C}} E \left(Π\right) \\ = arg {min}_{Π ∈ {P}_{C}} αg \left(Π\right) + h \left(Π\right) \\ = arg {min}_{Π ∈ {P}_{C}} {\left‖{ΠV}_{x}-{V}_{Y}\right‖}^{2} + {\left‖{ΠD}_{x}-{D}_{Y}Π\right‖}^{2} \\ = {argmax}_{Π ∈ {P}_{C}} \left〈Π,{αV}_{Y}{V}_{X}^{⊤}+{D}_{Y}{ΠD}_{X}\right〉 .\end{matrix}$ The optimization problem is solved by iteratively applying the Hungarian algorithm to the gradient of *E*, which is given by: $∇ E = {V}_{Y} {V}_{X}^{⊤} + {D}_{Y} {ΠD}_{X}$ yielding the step: ${Π}^{k + 1} = {argmax}_{Π ∈ {P}_{C}} \left〈Π,α{V}_{Y}{V}_{X}^{⊤}+{D}_{Y}{Π}^{k}{D}_{X}\right〉 .$ For initialization only, pointwise descriptors are used which results in: ${Π}^{0} = {argmax}_{Π ∈ {P}_{C}} \left〈Π,{V}_{Y}{V}_{X}^{⊤}\right〉 .$

### Classification

Although a position change detector can work on individual QRS-complexes, to improve the robustness it is assumed that the basket catheter does not move during a recording, and thus all QRS-complexes should be the same. Therefore, QRS-complexes are averaged: ${V}_{{X}_{avg}} = {\sum }_{i = 0}^{N} {V}_{{X}_{i}} and {V}_{{Y}_{avg}} = {\sum }_{i = 0}^{M} {V}_{{Y}_{i}} ,$ with **V**_{**X***ᵢ*} being the ith of N QRS-complexes of the basket placed in **X,** and **V**_{**Y***ᵢ*} being the ith of *M* QRS-complexes of the basket placed in **Y.** Usually *N != M* since the heart rate can change during the procedure.

After convergence of the optimization problem, an optimal **Π*** is obtained, which represents the minimal cost of aligning pairwise and pointwise descriptors. For classification, and whether or not recordings are done in the same position, *tr*(Π***) is used. If recordings are done in the same position, **Π*** should result in the identity matrix, and thus *tr(*Π**)* = C should hold. Experiments show that although recordings may be done in the same position, due to noise, *tr(*Π**)* = C is not always true. Therefore a threshold *γ* can be determined experimentally resulting in a discrimination rule: $\left\{\begin{matrix}same position , & tr \left(Π*\right) \geq γ \\ different position , & tr \left(Π*\right) < γ .\end{matrix}\right.$

### Relative Positioning

A concept for position change detection is described above. That approach uses the potential differences of multiple electrodes to gain spatial insights. However, classification is only possible if recordings are done in the same position. In addition, the method described it is only able to match bijectively, and thus recordings with the basket catheters not fully overlapping, but only partially, result in poor correspondence mapping. Therefore, and considering Fig. 9, which shows not only the consistency of QRS-complexes for a certain location, but also shows that the space of QRS-complexes is continuous (see also Fig. 10C). This means that a slight electrode displacement can also result in a slight change of the QRS-complex, while a larger electrode displacement also results in a greater change in the QRS-complex.

This motivates the idea of learning the function $V \left(x, t\right) ∈ {ℝ}^{T}$ to embed a QRS-complex in a three dimensional space, representing the position where the electrode was placed from which was recorded the given QRS-complex. Phrasing it differently, given the potential differences **V_{X}** what are the three dimensional coordinates **X?**

Expanding this idea to the full basket catheter results in the function $V \left(X, t\right) ∈ {ℝ}^{C \times T}$, where **X** is the three dimensional coordinate of the electrodes.

As described above, autoencoders exploit the idea that data concentrates around a low-dimensional manifold. For the purpose of learning the function V(X,t), the data are the QRS-complexes and the low-dimensional manifold is the three dimensional spatial location. Thus, the goal is to learn the function mapping from QRS-complexes to three dimensional coordinates. By embedding multiple QRS-complexes from multiple positions into this embedding space, a navigation system can be built which visualizes the multiple positions of basket catheters and their relative locations with respect to one another.

### Constrained Autoencoders Learning the QRS-Complex Manifold

Autoencoders can learn to compress a QRS-complex into a low dimensional representation. Constraining the low dimensional representation, the learned embedding space can retrieve spatial insights. Since there are no information available about the global positioning, the goal is to learn a relative positioning of QRS-complexes in the embedding space. To this end, and in one embodiment a constrained autoencoder may be used, where the embedding space is constrained by the properties of the employed basket catheter. Therefore, and as described above, pairwise descriptors representing the Euclidean distance of electrodes may be utilized in the loss term.

The loss term *L* may be described as: $L = {L}_{v} + {βL}_{z} ,$ with *Lᵥ* being the reconstruction loss of the QRS-complex and *L_{z}* being the embedding loss. *β* is the weighting balancing the two losses.

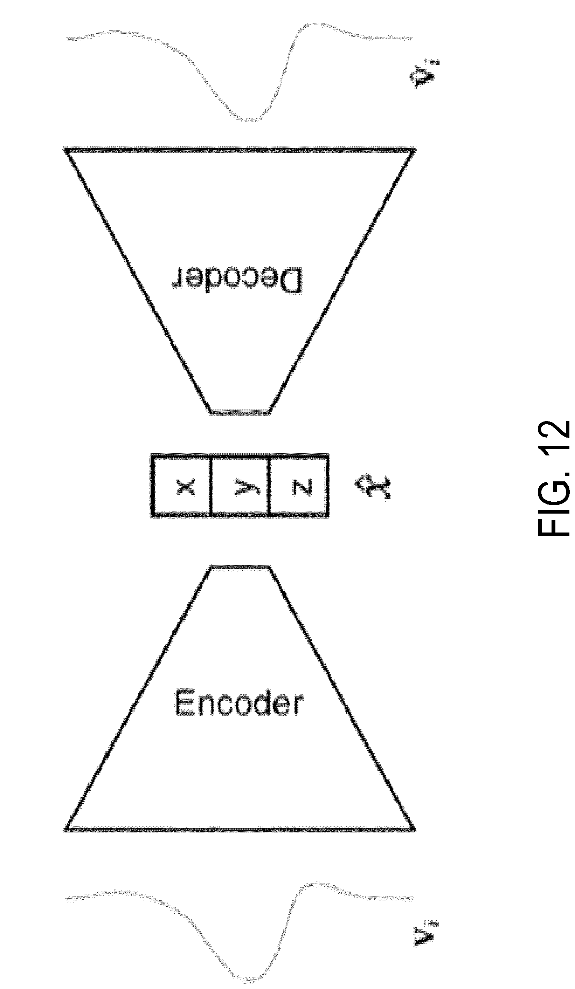

The autoencoder has a fully connected encoder and decoder. The input is the full QRS-complex with its *T* timepoints, and accordingly the output is also *T* timepoints *(T* is usually 100). The code is three-dimensional representing the *x, y* and *z* resulting in the coordinate *(x, y, z).* One embodiment of such a schematic architecture is shown in Fig. 12, where the autoencoder is configured to learn the manifold of the QRS-complexes. The input is a single QRS-complex v_i, compressed into a three-dimensional representation x"and then reconstructed to (v_i )^. The code x^corresponds to the coordinate of the QRS-complex where v_i was recorded.

The reconstruction loss *Lᵥ* may be defined as: ${L}_{v} \left({V}_{X}{\hat{V}}_{X}\right) = \frac{1}{C} {\sum }_{i = 1}^{C} {\left({v}_{i}-{\hat{v}}_{i}\right)}^{2} ,$ which is the mean-squared-error loss, with **V_{X}** being the QRS-complexes at the location **X** as input and output of the network, which is the reconstruction of the input **V_{X},** as **V̂_{X}**.

Since the pairwise descriptors, i.e., the spatial constraints of electrode distances, can only be included using multiple QRS-complexes, according to one embodiment a suitable architecture is a Siamese autoencoder having shared weights in the encoder and decoder.

For simplicity in loss calculation and back-propagation, in one embodiment the autoencoder is a C-fold Siamese autoencoder, where C is the number of electrodes. This simplification allows the loss calculation and backpropagation to be calculated for a full QRS-complex across all C electrodes. By this modification, the embedding space of the autoencoder is still three dimensional, but with C projections at once.

As a shape to constrain projections **X,** a spherical undeformed basket catheter may be used. To apply this constraint to the embedding space, a loss *L_{z}* is introduced, which compares the distance matrices of the spherical undeformed basket catheter ${D}_{sphere} ∈ {ℝ}^{c \times C}$ (see fig. 6.6) with the estimates ${D}_{\hat{X}} ∈ {ℝ}^{C \times C}$. This results in the formulation of *L_{z}*: ${L}_{z} \left({D}_{sphere}, {D}_{\hat{X}}\right) = {\left‖{D}_{sphere}-{D}_{\hat{X}}\right‖}^{2} .$

The assumption of a undeformed basket catheter is again a simplification, since the actual deformation of a basket is not known. However, since *L_{z}* is only one part of the loss term L the assumption is that the other loss terms will result in deformations of the basket to achieve global minimal cost.

Not all deformations of a basket catheter are equally likely. Including a proper physical model would be ideal. Unfortunately, this is not feasible in many circumstances, which is why the embedding loss *L_{z}* may be enhanced by a weighting matrix $M ∈ {ℝ}^{C \times C}$, which weights each electrode pair according to the likelihood of deviations. The loss term *L*_{z} results in: ${L}_{z} \left({D}_{sphere}, {D}_{\hat{X}}\right) = M ∘ {\left‖{D}_{sphere}-{D}_{\hat{X}}\right‖}^{2} .$

The weighting matrix **M** is constructed rudimentary by the following principles:
1. Deformations of further distanced electrodes require less force than those of nearby electrodes.
2. Deformations of neighboring electrodes along a spline are less likely due to the stiffness of the splines.
3. Deformations of electrodes on the 1st and 8th electrode rings are also less likely due to the stiffness of the basket catheter at the tip and pole.

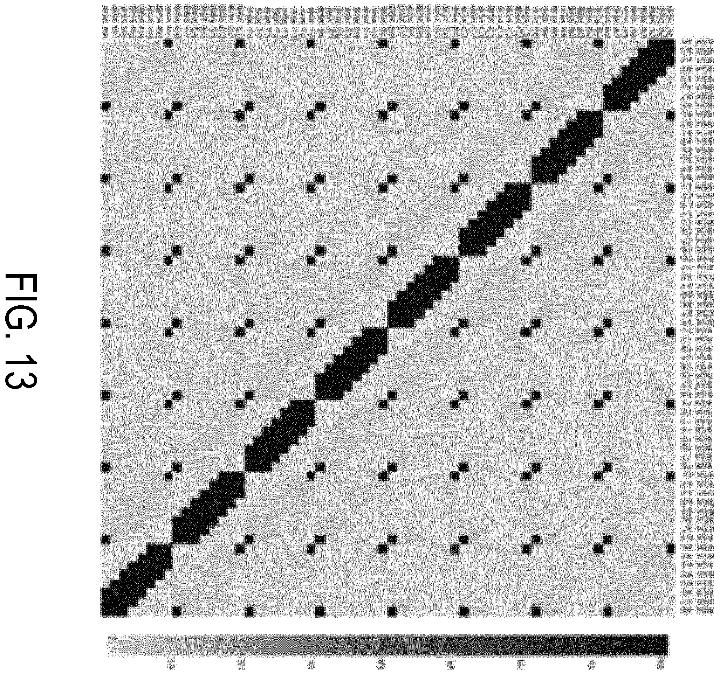

These three principles do not describe the physical properties of the basket catheter. The resulting weighting matrix from these principles is only an approximation, which has to be further optimized or extended to an actual physical model of the basket. However, in some embodiments the resulting weighting matrix **M** shown in Fig. 13 can be considered as a hyperparameter. A weighting matrix **M** was constructed by approximating the required forces to deform a basket catheter based on the foregoing three principles.

Fig. 13 shows a weighting matrix M for the loss L_{z}. Each entry in Fig. 13 specifies the weight in the loss for an electrode pair. Neighboring electrodes along a spline and electrodes on the 1st and 8th ring have a very high weight. All other electrodes have a weight corresponding to inverted distances, i.e., close electrodes have a high weight, and further distanced electrodes have a low weight.

Since the goal is to retrieve relative positioning of the electrodes for the whole recording instead of for only individual QRS-complexes, again the fact that the basket catheter does not move over the full duration of a recording is utilized to increase the robustness of the network against noise. Therefore, instead of building the distance matrix **D_{X̂}** from the individual estimates **X̂***ᵢ* the distance matrix is built using the average position estimates **X*****X̂**_{avg}*.
The final loss *L_{z}* is then: ${L}_{z} \left({D}_{sphere}, {D}_{{\hat{X}}_{avg}}\right) = M ∘ {\left‖{D}_{sphere}-{D}_{{\hat{X}}_{avg}}\right‖}^{2} .$

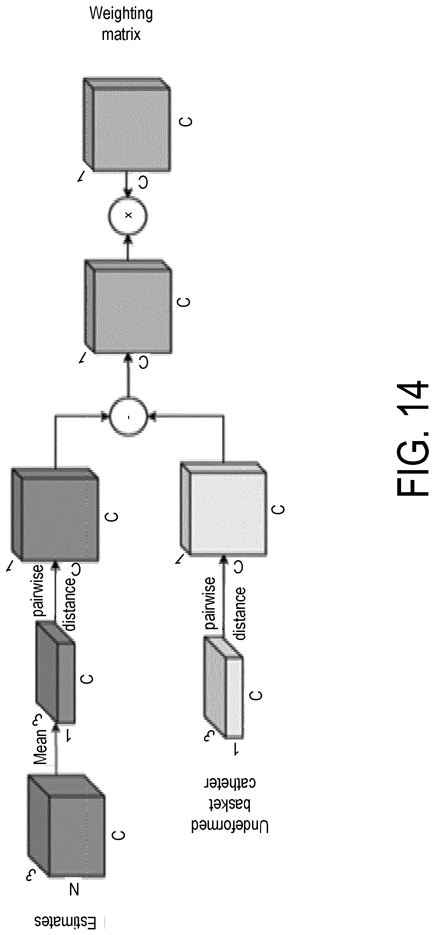

One embodiment of a computation graph and flow diagram for L_{z} is illustrated in Fig. 14, where the loss function *L_{z}* is computed from the estimates of undeformed basket catheter coordinates, and distance matrices are computed, subtracted and then multiplied with their respective entries in the weighting matrix. The network is trained on all recordings of a patient in an unsupervised manner. To estimate the positions of the electrode for a whole recording during inference **X̂**_{avg} may be used.

### Atrium Classifier and Experiment Setup

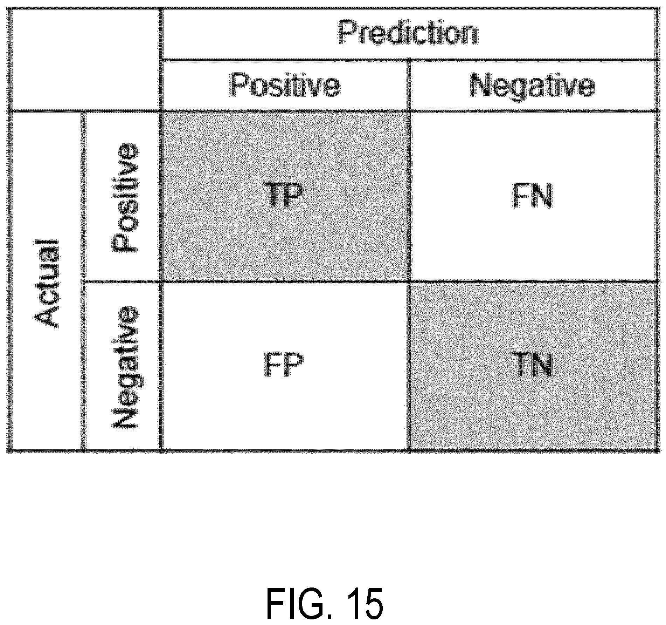

In one embodiment, an atrium classifier is a 3D convolutional neural network comprising two convolutional layers, each with an ReLU activation function followed by a max pooling operation. In such an embodiment, the last layer may be a fully connected dense layer with two units, representing left and right atrium one-hot encoded. The output is normalized using sigmoid as an activation function. Such a network architecture is depicted in Fig. 15 and in Table 3 below.

Fig. 15 shows one embodiment of a Confusion Matrix, and how TP, FN, FP and TN can be defined. Each row represents one layer. After a convolution with an activation function, a max pooling operation is followed. The last layer is a fully connected dense layer with two units, representing left and right atrium one-hot encoded. Same padding indicates a zero padding, so that the input shape is the same as the output shape. Valid padding indicates no additional padding.

The input to the network is the average QRS-complex per recording per electrode. For optimization Adam with a learning rate of 1e-3 was used. Training was done in a supervised manner until convergence occurred using an early stopping of 20 epochs. Due to the limited amount of training data, three models were trained using 3-fold cross validation on the training set.

For evaluation on the held out test set, an ensemble of the three models was used. Therefore, the one-hot encoded output of the three models was averaged, resulting in a single one-hot encoding, indicating the classified atrium.

### Performance Metrics

Since the task of atrium classification is binary, accuracy is used as a performance metric. Accuracy may be defined as: $Accuracy = \frac{TP + TN}{TP + TN + FP + FN}$ with TP, FP, TN and FN (the confusion matrix in Fig. 15 describes the respective meanings of these terms).

**Table 3**

| Name | Kernel size | Stride | Padding | Activation fct. | Output shape | Parameters |
|---|---|---|---|---|---|---|
| Conv3D | 4×3×3×5 | 1×1×2 | Same | ReLU | 8×8×49×4 | 184 |
| MaxPool3D | 2×2×3 | | | | 4×4×16×4 | |
| Conv3D | 2×1×1×5 | 1×1×2 | Valid | ReLU | 4×4×6×2 | 42 |
| MaxPool3D | 2×2×3 | | | | 2×2×2×2 | |
| Dense | 2 | | | Sigmoid | 2 | 34 |

### Evaluation

Table 4 below shows the results of an atrium classifier applied to the test set. Each row depicts a model's accuracy for a single patient. The last row is the model's average accuracy on the entire test set. The second to last column is the accuracy using the ensemble of the three models. The last column is the average performance of all three models (without the ensemble) for each patient. There are three models since the training was done using 3-fold cross validation.

**Table 4**

| Patient | Model #1 | Model #2 | Model #3 | Ensemble | Patient avg. |
|---|---|---|---|---|---|
| 02-004 | 0.774 | 1.000 | 0.968 | 0.923 | 0.914 ± 0.122 |
| 02-008 | 0.600 | 0.800 | 0.700 | 0.730 | 0.700 ± 0.100 |
| 02-012 | 0.333 | 0.750 | 0.750 | 0.622 | 0.611 + 0.241 |
| 03-019 | 1.000 | 0.714 | 0.714 | 0.840 | 0.810 ± 0.165 |
| Model avg. | 0.677 ± 0.282 | 0.816 ± 0.128 | 0.783 ± 0.125 | **0.779** ± **0.131** | **0.759** ± **0.185** |

Table 4 shows the accuracy of the held out test set for the three models. The average accuracy on the test set is 0.759 with a standard deviation of ± 0.185. Ensemble predictions using all three models result in slightly better accuracy than the average accuracy of all models (0.779 vs 0.759). Also the standard deviation is smaller using the ensemble (0.185 vs 0.1.31). Model #1 has the highest standard deviation with ± 0.282 resulting from an accuracy of only 0.333 on patient 02-012 and 1.000 on patient 03-019. For the other two models the standard deviation is less than half of Model #1.
Overall Model #2 and Model #3 individually have a higher accuracy and lower standard deviation than using the Ensemble.

### Position Change Detector Experiment Setup

The position change detector is evaluated using the foregoing dataset. For each patient, all recordings are compared with one another. The same position and position change were defined by the physician during the procedure.

The threshold *γ* from for binary classification was determined by the threshold maximizing *TPR - FPR,* with TPR being the true positive rate and FPR the false positive rate, using the training set. This threshold is then used to evaluate the position change detector on the held out test set: $TPR = Recall = \frac{TP}{TP + FN}$ $FPR = \frac{FP}{FP + TN}$

### Performance Metrics

The performance of the position change detector is assessed by the precision, recall and F₁-Score. The F₁-Score (also known as the Dice similarity coefficient) is the harmonic mean of precision and recall. Its highest possible value is 1.0, indicating perfect precision and recall. The lowest possible value is 0, if either precision or recall is 0: $Precision = \frac{TP}{TP + FP}$ ${F}_{1} = 2 ⋅ \frac{Precision ⋅ Recall}{Precision + Recall}$

### Evaluation

First the threshold *γ* is determined using the training set. The receiver operating characteristic (ROC) curve is a plot illustrating the performance of a binary classifier while the threshold is varied. Therefore, from the ROC curve (see Fig. 16A) the different thresholds are analyzed resulting in the maximal *TPR - FPR* score at 14 (see Fig. 16B), i.e. *γ* = 14.

In Fig. 16A, an ROC Curve for the training set is shown, where along the x-axis the FPR is displayed, and along the y-axis the TPR is shown. The dashed line indicates a random classifier. In Fig. 16A, the AUC is colored in blue. In Fig. 16B, the hyperparameter optimization for γ is shown. The x-axis depicts the different thresholds, while the y-axis depicts the TPR-FPR. The maximum TPR-FPR is achieved at a threshold of 14, and thus γ = 14 (red line).

After determination of the threshold y, the position change detector was evaluated in the held out test set using the introduced performance metrics. This resulted in an average precision of 0.996, a recall of 0.972, and an F₁-Score of 0.984. The per patient scores are shown in Table 5, where the results of the same position detector in the test set are shown. Each row depicts a single patient with the achieved performance. The last row in Table 5 is the average performance across the entire test set.

In Fig. 16C, a histogram of identity correspondences is shown, where a margin between same position and position change identity correspondences is displayed. Along the x-axis, the number of identity correspondences is shown, while along the y-axis the frequency is shown. The y-axis is in log scale. In Fig. 16C, blue bars correspond to comparisons of recordings in the same position, while orange bars correspond to recordings in different positions.

**Table 5**

| Patient | Precision | Recall | F₁-Score |
|---|---|---|---|
| 02-004 | 0.984 | 0.974 | 0.979 |
| 02-008 | 1.000 | 0.998 | 0.999 |
| 02-012 | 1.000 | 1.000 | 1.000 |
| 03-019 | 1.000 | 0.915 | 0.956 |
| Avg. | 0.996 | 0.972 | 0.984 |

### Relative Positioning and Experimental Setup

The encoder and decoder of the architecture for relative positioning algorithm are fully connected networks with ReLU activation functions. An architecture of a relative positioning autoencoder with such parameters is illustrated in Table 6. All layers are fully connected with an ReLU activation function. Each column represents a layer with the number of neurons (second to the last row) and the number of parameters (last row). The encoder and decoder each comprise three layers.

**Table 6**

| | Layers | | | | | |
|---|---|---|---|---|---|---|
| | Encoder | | | Decoder | | |
| Neurons | 50 | 25 | 3 | 25 | 50 | 100 |
| Parameters | 5050 | 1275 | 78 | 100 | 1300 | 5100 |

For each patient a separate model was trained with all recordings at once. One batch consists of multiple data points, while one data point consists of all QRS-complexes across all electrodes for a single recording. For optimization Adam was used with a learning rate of 1e-3. The network was trained until convergence using early stopping with 100 epochs. Since the training was unsupervised, all training data was used during the training process.

### Performance Metrics

For quantitative performance assessment, multiple metrics were used. For global comparison of the estimates against the reconstructed ground truth, they were first rigidly aligned using the Kabsch algorithm(). Afterwards the average distance between corresponding electrodes was measured.
The resulting metric is defined as: $D = \frac{1}{R ∗ C} {\sum }_{j}^{R} \left‖{\hat{X}}_{{avg}_{j}}-{X}_{{gt}_{j}}\right‖ ,$ with R being the number of recordings, C the number of electrodes, **X̂***_{avgⱼ}* the electrode position estimates of the *j*-th recording and **X***_{gtⱼ}* the corresponding reconstruction of the actually used navigation system.

For discrimination and better understanding of where the average distance error was resulting from, two additional metrics were used. For assessment of the global positioning error of the catheter, the center points of the estimates and reconstructed ground truth were compared. The center point of the catheter was the mean 3D coordinate of all electrodes. This global positioning error *D_{center}* is defined by: ${D}_{center} = \frac{1}{R} {\sum }_{j}^{R} \left‖{\hat{\overline{X}}}_{{avg}_{j}}-{\overline{X}}_{{gt}_{j}}\right‖$ with ${\hat{\overline{X}}}_{{avg}_{j}} ∈ {ℝ}^{3}$ being the center point of all electrodes of **X̂***_{avgⱼ}* and ${\overline{X}}_{{gt}_{j}} ∈ {ℝ}^{3}$ being the center point of all electrodes of **X***_{gtⱼ}*.

For assessment of the deformation error, a rigid alignment was done individually for each basket catheter, i.e., only locally. As a result, the global positing/rotation error was filtered out, permitting assessment of the deformation error *D_{deformation}.* The metric for this deformation error *D_{deformation}* was the same as in the equation for D above.

### Evaluation

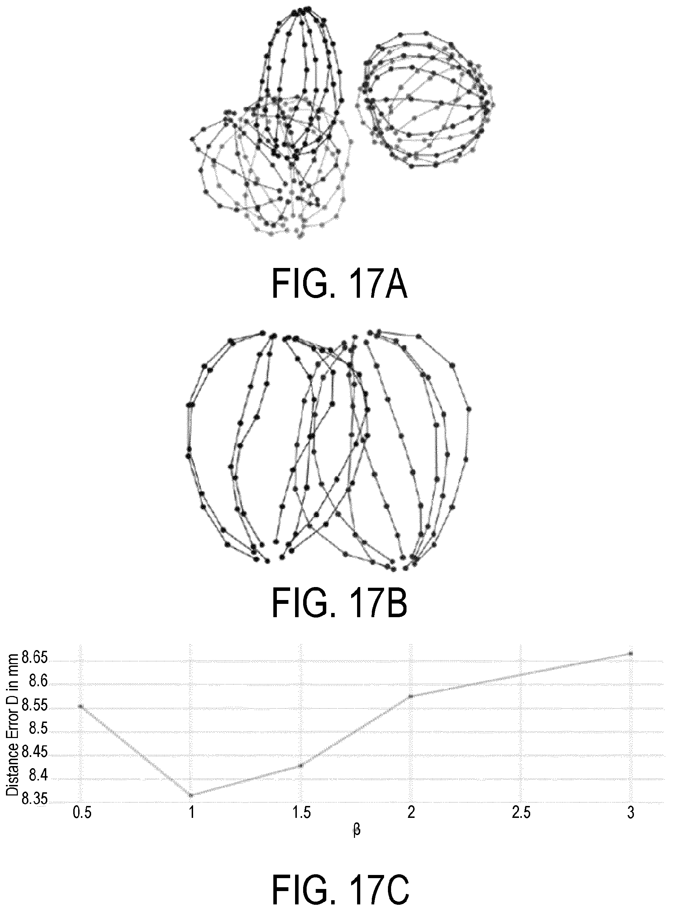

The first conducted experiment was aimed at learning on all recordings of a patient at once. A qualitative result is shown in Fig. 17A, where atrial collapse of relative positioning is illustrated, the right atrium is shown in blue, and the left atrium is shown in red. The two positions are overlapped, which is anatomically impossible. The recordings from the two atria were separated (which is in line with the hearts anatomy, since the two atria are separated by the septal wall). However, for some patients there is an overlap of the estimated positions from the left and right atrium (see Fig. 17B), which is anatomically not possible. In Fig. 17B, atrial collapse of relative positioning is also shown, where again the right atrium is shown in blue, and the left atrium is shown in red. The two positions again are overlapped, which is anatomically impossible.

As a result, the task of relative positioning was split according to each atrium, and thus a separate network was trained per atrium per patient. The information about each atrium in which an intracardiac EGM was recorded was taken from the atrium classifier described above. However, for the purpose of isolating evaluation of the relative positioning algorithm, this information was taken from the dataset itself, and not inferred from the atrium classifier.

The weighting between the two loss terms *Lᵥ* and *L_{z}* was encoded by the β determined experimentally from the training set. A minimal distance error D was achieved with a *β* of 1 shown in Fig. 17C, where the x-axis depicts the different choices of beta, and the y-axis depicts the average distance error of the estimates against the ground truth of the training/validation set. The smallest error was obtained at β = 1.

Evaluating the relative positioning algorithm applied to the test set with the determined *β* results in the performance metrics shown in Table 7. The different columns in Table 7 show the different performance metrics. The first row in Table 7 shows the performance in the left atrium, and the second row shows the performance in the right atrium. The last row shows the average performance across both atria. Units are shown in mm.

**Table 7**

| | D | *D_{center}* | *D_{deformation}* |
|---|---|---|---|
| Left atrium | 7.68 ± 3.14 | 3.26 ± 1.52 | 6.05 ± 2.66 |
| Right atrium | 9.90 ± 3.90 | 7.43 ± 2.69 | 7.51 ± 3.37 |
| Avg. | 8.80 ± 3.53 | 5.34 ± 2.10 | 6.78 ± 3.01 |

Continuing to refer to Table 7, the average distance D is 8.80 mm with ± 3.53 mm standard deviation. While for the right atrium D is larger than for the left atrium, the standard deviation (9.90 ± 3.90 vs. 7.68 ± 3.14) must also be considered. The positioning error for the center of the catheters *D_{center}* is 5.34 ± 2.10 mm. This error for the right atrium is more than twice that associated with the left atrium (7.43 ± 2.69 mm vs. 3.26 ± 1.52 mm). The metric for assessing the deformation *D_{deformation}* has the smallest discrepancy between the left atrium (6.05 ± 2.66 mm) and the right atrium (7.51 ± 3.37 mm), resulting in an average performance of 6.78 ± 3.01 mm.

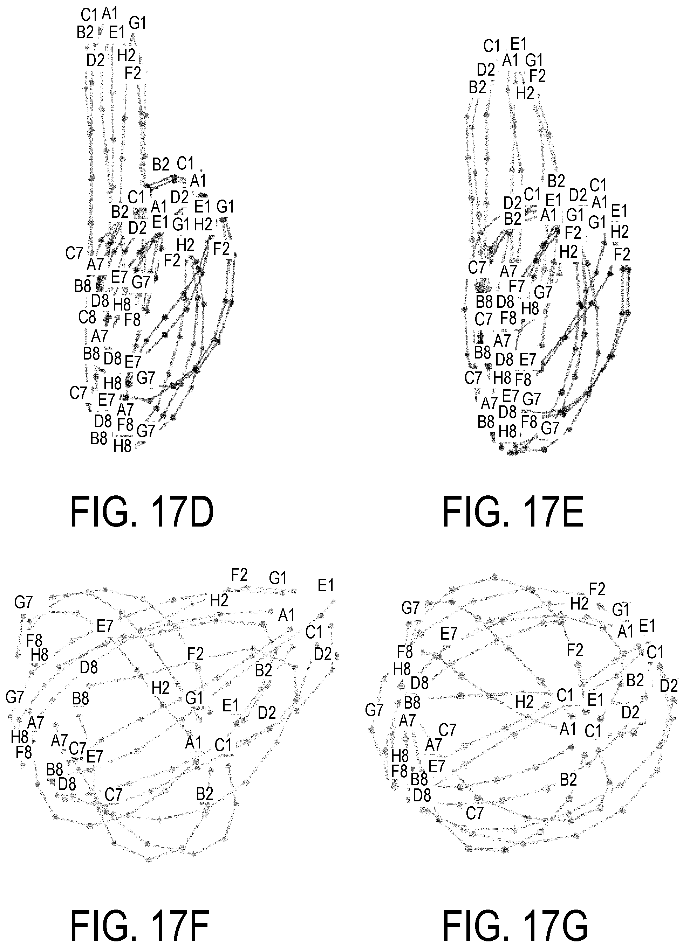

A qualitative comparison of ground truth reconstructions against estimates is shown in Figs. 17D and 17E for the right atrium, and in Figs. 17F and 17G for the left atrium. In each such comparison, the rotation, translation and scaling is the same. In Figs. 17D and 17E, qualitative comparisons of three basket catheter positions in the right atrium is shown. Ground truth is shown in Fig. 17D, while estimates are shown in Fig. 17E. Both ground truth and estimates are rigidly aligned in Figs. 17D and 17E, where the same color indicates the same recording. From electrode names, splines can be inferred. The undeformed catheter has a diameter of 50 mm.

In Figs. 17F and 17G, a qualitative comparison of two basket catheter positions in the left atrium is shown. Ground truth is shown in Fig. 17F, while estimates are shown in Fig. 17G. Ground truth and estimates are rigidly aligned. The same color indicates the same recording. From electrode names, splines can be inferred. The undeformed catheter has a diameter of 50 mm.

### Atrium Classifier Results

In one tested embodiment, the atrium classifier was determined to have an accuracy of 0.759 ± 0.185 when applied to the test set, a reasonable performance. Using an ensemble of the three models improved the accuracy to 0.779 ± 0.131. The reason for this improvement might be the utilization of the individual models confidence. While for the average accuracy the binarized prediction is averaged, within the ensemble the probability values are averaged, thus leveraging the confidence of the different models.

### Limitations

The foregoing-described performance metrics suggest that the QRS-complexes in the right and in the left atrium may not always be clearly distinguishable across patients. This might be due to contradicting QRS-complex patterns across patients, meaning that similar QRS-complexes are observed not only in the left atrium of one patient, but also in the right atrium of another patient. It might just be the case that the dataset employed was too small to learn the variations of QRS-complexes across patients. With more training data this problem can likely be solved. This hypothesis is especially supported by the sensitivity of the models' performance applied to the data set split described above. Further experiments on larger datasets will be helpful to investigate this hypothesis. However, the relative positioning algorithm showed that it was actually capable of separating left and right atrium recordings from a single patient (See Fig. 17A). Unfortunately, this does not hold true for all patients (see Fig. 17B). Nevertheless, our results indicate that in at least some embodiments an atrium classification is workable when only a single patient is considered instead of multiple patients.

### Position Change Detector Results

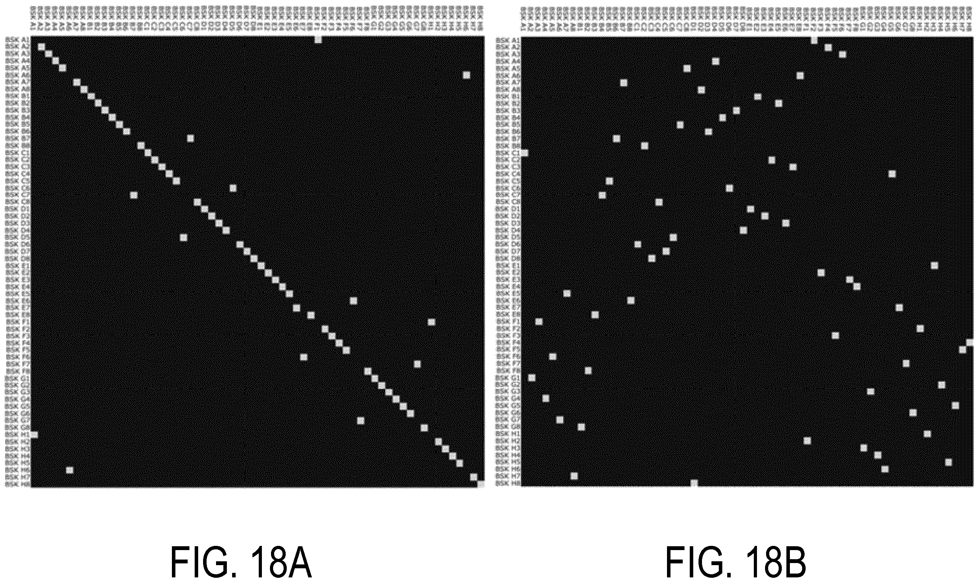

The position change detector described above with its high precision, recall and F₁-score shows that actual reduction to practice of reliable position change detection has been achieved. That there are almost no misclassifications and the margin between same position and position change in the histogram as regards identity correspondences (see Fig. 16C) can also be interpreted as a certainty measurement. Accordingly, the position change detector is of great benefit to a clinician. To justify the claim that reasonable correspondence matching can be distinguished from unreasonable correspondence matching, two different correspondence matches are shown in Fig. 18A (reasonable matching) and Fig. 18B (unreasonable matching). While in Fig. 18A most correspondences are along the diagonal (indicating good correspondence matching), only a few correspondences are situated along the diagonal in Fig. 18B (indicating poor correspondence matching). In Fig. 18A, *Π** results are shown for two different recordings in different positions. Yellow dots indicate correspondences. Most of the yellow dots are on the diagonal *(tr(Π*)* > *γ).* In Fig. 18B, *Π** results are shown for two different recordings in different positions. Yellow dots indicate correspondences. Most of the yellow dots are off the diagonal *(tr(Π*)* < *γ).*

### Relative Positioning Results

The performance of the embodiments described and disclosed herein using a relative positioning algorithm, when training on all recordings from both atria at once, suffers from some deficiencies due to anatomically impossible overlapping positioning of the two atria. See Fig. 17B. However, results from training using data from two atria separately (see Table 7) indicate the general capability to navigate and position using purely biosignal based signals. Also, a qualitative visual comparison between reconstructions from the actual navigation system used and those from the relative positioning algorithm are convincing. The reason for a higher electrode difference *D* and *D_{center}* in the right atrium as opposed to the left atrium may lie in the space between the two atria. In the left atrium, the overlap between individual basket positions is usually high, thus resulting in a high information density. In the right atrium, the catheters are also placed in the veins (e.g., the superior vena cava and the inferior vena cava), which results in a substantially smaller overlap (compared to the left atrium) between different catheter positions. Furthermore, and especially in the superior vena cava, the distance to the ventricles is the largest compared to other positions, which results in the lowest signal to noise ratio, since the ventricles are the origin of the signals employed for navigation. The better performance of the relative positioning algorithm in the left atrium compared to the right atrium with respect to the deformation error *D_{deformation}* may be due to the stronger deformations applied to the catheter when placed in the right atrium. The reason for such stronger deformations is again the placement of the catheter in the veins associated with the right atrium, while in the left atrium the catheter remains more spherical. The performance data described above show that relative positioning information can be extracted successfully from intracardiac EGMs.

### Some Limitations

As described above, the accuracy of the ground truth, which is reconstructed from the navigation system used during data acquisition, is not fully known. However, it is known that the error is at least 0.46 ± 0.17 mm. This has to be considered when evaluating quantitative performance.

Since the introduced algorithm only works with relative positioning, global positioning and thus actual catheter spatial orientation is unknown (i.e., is it not known where up, down, left, right, front and back of the catheter are positioned). To constrain the representation space during training, a prior shape in the embedding space may be used. The prior shape of an undeformed basket catheter can be applied using a pairwise distance matrix. From there, the problem arises that a 3D shape and its point-mirrored 3D shape can result in the same pairwise distance matrix. This itself is not a problem since the basket catheter has a specific ordering of splines (alphabetically clockwise on the 1st electrode ring, viewed from the outside), from which it can be determined whether the catheter electrodes are in the correct order or not. This ordering is described hereafter as "winding."

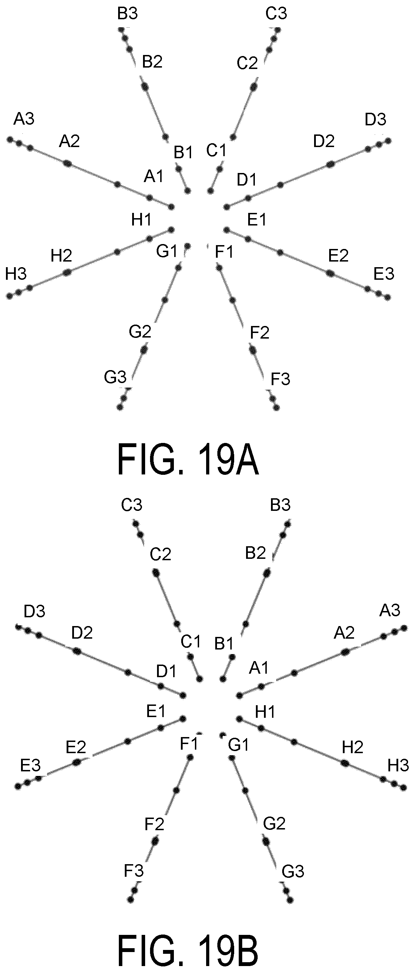

An example of correct basket catheter electrode winding is shown in Fig. 19A, and an example of incorrect catheter electrode winding is shown in Fig. 19B. Incorrect winding results when the ordering of splines cannot be achieved with a real basket catheter, but is not constrained by the relative positioning algorithm. In the case where all basket catheter estimates are wound incorrectly, the whole space can just be point-mirrored in a postprocessing step, resulting in a correct winding. This is possible since the algorithm works with relative positioning and not absolute positioning, and thus global rotation and translation are not considered by the algorithm. A problem arises when multiple basket catheter estimates have different windings. A point mirroring of the space would fix the winding of those previously incorrectly wound, but also vice versa, resulting again in estimates with different windings.

The prior shape used for training the network is a perfect sphere. This is not ideal since the basket catheters can be deformed substantially. However, with a perfect spherical basket catheter as a prior shape, estimates tend to be more spherical than they actually are. A proper model of deformation possible or likely in a basket catheter can be of benefit to increase determinations of the likelihood of basket catheter deformation.

Along with the previous point, in some embodiments the weighting matrix for loss which encodes the likelihood of deformations can be rudimentary, as it may be purely based on expert knowledge, and not using a proper experimental approach to determine the actual likelihood of deformations.

The training process of the relative positioning algorithm utilizes all recordings of the same atrium at the same time. While such an approach can be used to investigate retrospective procedures, problems may occur when such an approach is used during an ongoing procedure, since the recordings are done sequentially. The relative positioning algorithm can be modified, if necessary, to embed a new recording which was not used during the training process. However, if the position of the new recording has only a small overlap with previous recording positions, errors may occur since the embedding space was not properly constrained. Thus, with each new recording such an approach would result in a retraining (or finetuning) of the whole network.

### Some Conclusions

Bringing all the methods disclosed and described herein together permits a purely biosignal based intracardiac navigation system to be constructed. The atrium classifier is used to classify the atrium in which a recording is made. Then, the position change detector identifies if another recording has already been carried out done in the same catheter position. If so, the current representation space of the relative positioning algorithm is refined by pairing the new recording with recordings previously made in the same catheter position. More importantly, if there was no other recording in the same position previously, the relative positioning algorithm can embed the new recording in the embedding space either directly or by retraining the network as described above.

Here we describe the first purely biosignal based intracardiac catheter navigation and positioning system. To this end, three methods are described and disclosed, all utilizing the QRS-complexes of intracardiac EGMs. First, a convolutional neural network is presented which is capable of classifying in which atrium a recording has been made. Then a position change detector classifies and determines whether two recordings have been made in the same position by comparing their QRS-complexes. Next, the relative positioning algorithm embeds the QRS-complexes of the intracardiac EGMs into a 3D representation space, which represents the positions of the basket catheter during the recording of an intracardiac EGM. The described methods clearly show that positioning information can be extracted successfully from an intracardiac EGMs.

For future work, most interesting is probably to further improve the relative positioning algorithm. There is a need for a dataset with a higher accuracy than the one presented and described herein so that the performance of the relative positioning algorithm can be quantified with greater precision. This can be achieved using realistic simulations of ventricular signals. Another interesting direction for future work is to extract global orientation information from intracardiac EGMs. The amplitude of the QRS-complex decays over the distance of the ventricles from the origin of the signals therein. By utilizing this fact, global orientation information (up and down direction) can be extracted from an EGM. The prior shape and weighting matrix used in the relative positioning algorithm can also be improved. Experimental retrieval of the actual physical deformation properties of each type and model of basket catheters maybe important to provide improved catheter deformation estimates. The relative positioning problem has similarities to the one of SLAM. Including prior motion smoothness from this field of research would be interesting for a real-time relative positioning algorithm.

### One Embodiment of a Method Determine the Locations of Electrodes Inside a Patient's Heart Using Biosignals

According to one embodiment of a method of determining the locations of electrodes inside a patient's heart using biosignals, at least some of the following steps can be carried out by system 100 and computing device 300:
- Input: Raw electrograms (EGMs) and body surface electrocardiograms (ECGs): **V_{c}**(t) where c stands for the respective channel and t for time
- Apply preprocessing (high-pass filter, notch filter) to **V_{c}**(t) as described and disclosed herein;
- Detect QRST complex times [t₁, t₂, t₃, ... tₙ] from body surface ECG synchronized on the exact peak of the R wave as described and disclosed herein and as described and disclosed in the '249 and '605 patent applications to Ten Brink et al.
- For each of these times tᵢ, define windows around the R wave (from about 50 ms before peak to about 50 ms after peak): w₁, w₂, w₃, ... wₙ . These pre- and post-peak times can also be about 150 ms pre- and 1000 ms post-peak;
- For each body surface ECG channel, run a clustering algorithm using these windows, resulting in cluster memberships for each w₁, w₂, w₃, ... w_{N};
- Only consider windows wᵢ belonging to the largest cluster;
- For each such window, extract the respective signals ("isolated ventricular signals") from each EGM channel, resulting in a set of QRST morphologies per channel and per heart beat V_{c}(w₁, w₂, w₃, ... wₙ) (not considering low dominance morphologies, which belong to other clusters). This is equivalent to **Vₓ** in ${V}_{X} ∈ {ℝ}^{C \times T}$
- Define an auto-encoder neural network as described and disclosed herein as regards constrained autoencoders learning the QRS-complex manifold:
   - Use an input layer of the same dimensions as Vₓ: N x c x 100 where N indexes the QRST window, c the channel and 100 is the length of a window
   - Use layers and neurons according to Table 6 above;
   - Use a bottle neck ("code") X̂ of dimensions N x c x 3 (where 3 is chosen to represent 3 spatial coordinates);
   - Use the output layer with the same dimensions as the input layer;
   - Define the following cost functions according to *L= Lᵥ+βL_{z}:*
   - Reconstruction loss Lv = ∥ input - output ∥² according to ${L}_{v} \left({V}_{X}{\hat{V}}_{X}\right) = \frac{1}{C} {\sum }_{i = 1}^{C} {\left({v}_{i}-{\hat{v}}_{{i}_{}}\right)}^{2}$ ,
   - Use Regularization L_{Z} = sum of squares of pairwise distances between the estimated coordinates and those of a hypothetical undeformed basket catheter, with a point-wise weighting according to *L₂(Dₛₚₕₑᵣₑ,* D_{X̂}) = M ∘ ∥*Dₛₚₕₑᵣₑ* - D_{X̂}∥².
- Define an optimization schedule:
   - ADAM optimizer, learning rate 10⁻³, 100 epochs
- Iteratively apply the optimizer on the network so as to minimize the sum of Lᵥ and L_{z} by varying the parameters of the network
- In order to estimate the xyz coordinates of the c EGM electrodes for a given recording, average X̂ over the first axis (N) according to *L₂(Dₛₚₕₑᵣₑ,* D_{X̂}) = M ∘ ∥*Dₛₚₕₑᵣₑ* - D_{X̂}∥².

### Another Embodiment of a Method Determine the Locations of Electrodes Inside a Patient's Heart Using Biosignals

According to one embodiment of a method of determining at least one of a position and an orientation of an intra-cardiac electrophysiological (EP) mapping basket of an EP mapping catheter when the basket is disposed inside or near an atrium or other heart chamber or portion of a patient's heart, the EP mapping basket comprising a plurality of electrodes mounted on one or more arms or splines thereof, each electrode having a location or position on one or more splines or arms associated therewith, at least some of the following steps can be carried out system 100 and computing device 300:
- recording or acquiring a plurality of intra-cardiac signals inside or near the heart of the patient using the plurality of electrodes, a data acquisition or recording amplifier or device, and a computing device, the data acquisition or recording amplifier or device, and the computing device, being operably connected to the EP mapping catheter and the electrodes thereof, each of the recorded or acquired intra-cardiac signals having at least one electrode associated therewith;

▪ using the computing device, isolating or extracting ventricular signals from the at least some of the recorded or acquired intra-cardiac signals, and
▪ using the computing device, determining, using the extracted or isolated ventricular signals and the computing device, the locations of the electrodes inside or near the patient's atrium, heart chamber or other portion of the patient's heart that are associated with each isolated or extracted ventricular signal.

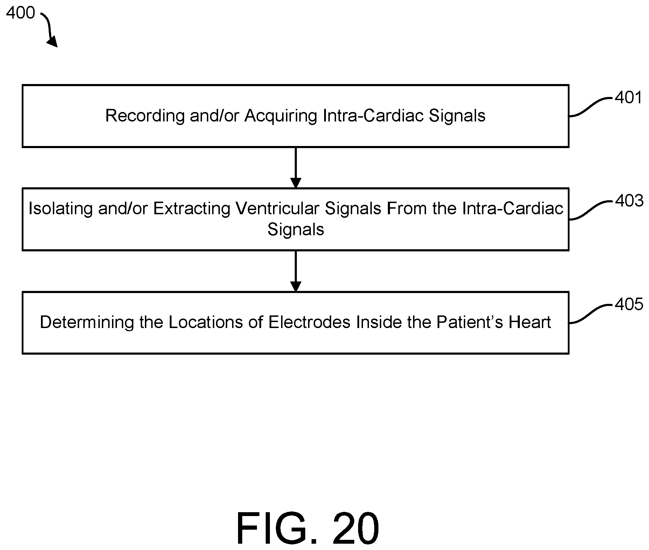

Fig. 20 illustrates one method 400 of determining the locations of electrodes inside a patient's heart. At step 401, intra-cardiac signals are recorded or acquired. At step 403, ventricular signals are isolated or extracted from the acquired intra-cardiac signals. At step 405, the locations of the electrodes employed to acquire the intra-cardiac signals are determined. Steps 401 through 405 are carried in accordance with the teachings and disclosures set forth herein.

### Additional Embodiments

In one embodiment, there is provided a method of determining at least one of a position and an orientation of an intra-cardiac electrophysiological (EP) mapping basket of an EP mapping catheter when the basket is disposed inside or near an atrium or other heart chamber or portion of a patient's heart, the EP mapping basket comprising a plurality of electrodes mounted on one or more arms or splines thereof, each electrode having a location or position on one or more splines or arms associated therewith. The method comprises recording or acquiring a plurality of intra-cardiac signals inside or near the heart of the patient using the plurality of electrodes, a data acquisition or recording amplifier or device, and a computing device, the data acquisition or recording amplifier or device, and the computing device, being operably connected to the EP mapping catheter and the electrodes thereof, each of the recorded or acquired intra-cardiac signals having at least one electrode associated therewith; using the computing device, isolating or extracting ventricular signals from the at least some of the recorded or acquired intra-cardiac signals; using the computing device, determining, using the extracted or isolated ventricular signals and the computing device, the locations of the electrodes inside or near the patient's atrium, heart chamber or other portion of the patient's heart that are associated with each isolated or extracted ventricular signal.

The foregoing method can further comprise one or more of the following: (i) navigating the intra-cardiac EP mapping basket of the EP mapping catheter inside or near the atrium or other heart chamber or portion of the patient's heart using the determined locations of the electrodes; (ii) wherein the isolated or extracted ventricular signals comprise at least one of QRS and QRST signals; (iii) recording or acquiring at least one body surface signal from the patient using at least one body surface electrode, the at least one body surface signal being acquired or recorded simultaneously or substantially simultaneously with the recorded or acquired intra-cardiac signals; (iv) using the computing device, isolating or extracting at least one ventricular signal from the recorded or acquired body surface signal, and determining, using the extracted or isolated intra-cardiac and at least one body surface ventricular signals and the computing device, the locations of the electrodes inside the patient's heart that are associated with each isolated or extracted ventricular signal; (v) wherein the isolated or extracted ventricular signals comprise QRS complexes; (vi) moving the basket inside the patient's atrium to different positions, and recording or acquiring intra-cardiac signals at each such position; (vii) using the computing device, removing low-frequency or other artefacts or noise from the recorded or acquired intra-cardiac and/or body surface signals; (viii) recording or acquiring a plurality of intra-cardiac signals for each electrode while the basket is in a given position within the patient's atrium, and, using the computing device, isolating or extracting a ventricular signal for each such intra-cardiac signal.; (ix) using the computing device, for each electrode an averaged ventricular signal is generated using the plurality of intra-cardiac signals; (x) wherein the averaged ventricular signal is an averaged QRS complex; (xi) using the computing device, determining changes in the three-dimensional locations and orientations of the basket and the electrodes thereof as the basket is moved around, in or near the patient's atrium; (xii) using the computing device, using position change detection methods in the computing device to detect basket catheter movements; (xiii) using the computing device, determining the three-dimensional coordinates ***X*** of each electrode for a given position and orientation of the basket within the patient's atrium; (xiv) using the computing device, embedding multiple QRS complexes associated with the ventricular signals acquired or recorded at multiple positions into an embedding space such that multiple positions of the basket at different locations and/or orientations in or near the patient's atrium can be visualized by a user on a screen or monitor operably connected to the computing device; (xv) wherein the multiple positions of the basket can be employed by the user to navigate the basket inside or near the patient's atrium; (xvi) providing a visual display to a user of the locations of the electrodes and/or basket inside or near the patient's atrium; (xvii) wherein an autoencoder is employed in the computing device to compress QRS complexes into low-dimensional representations thereof; (xviii) wherein the computing device is configured to learn relative positioning of QRS complexes in the embedding space; (xix) using the computing device and the isolated or extracted ventricular signals, at least one artificial neural network to determine the locations of the electrodes and/or basket inside or near the patient's atrium; (xx) wherein the at least one artificial neural network further comprises at least one feed forward network; (xxi) wherein the at least one artificial neural network further comprises at least one convolutional neural network; (xxii) wherein the at least one artificial neural network further comprises at least one autoencoder; and (xxiii) wherein the locations of the electrodes and/or basket inside or near the patient's atrium are provided in a visualization to a user in real time or near real time.

In another embodiment, there is provided a system configured to determine at least one of a position and an orientation of an intra-cardiac electrophysiological (EP) mapping basket of an EP mapping catheter when the basket is disposed inside or near an atrium or other heart chamber or portion of a patient's heart, the EP mapping basket comprising a plurality of electrodes mounted on one or more arms or splines thereof, each electrode having a location or position on one or more splines or arms associated therewith. The system comprises: (a) at least one computing device; (b) at least one data acquisition device operably connected to the at least one computing device or configured to provide as outputs therefrom at least one of electrical signals and at least one body surface electrogram signal; and (c) a display or monitor operably connected to the at least one computing device and configured to visually display to a user results generated by the at least one computing device; wherein the computing device comprises at least one non-transitory computer readable medium configured to store instructions executable by at least one processor to extract atrial signals from the plurality of electrical signals, the computing device being configured to: (i) receive or acquire, using the data acquisition device, the plurality of electrical signals using electrodes and at least one body surface electrogram signal using the at least one body surface electrode located on one or more body surfaces of the patient, wherein the electrical signals and the at least one body surface electrogram signal are acquired over the same or substantially the same first time window, the first time window being sufficiently long to capture a plurality of QRS complexes; (ii) recording or acquiring a plurality of intra-cardiac signals inside or near the heart of the patient using the plurality of electrodes, the data acquisition device, and the computing device, the data acquisition device and the computing device being operably connected to the EP mapping catheter and the electrodes thereof, each of the recorded or acquired intra-cardiac signals having at least one electrode associated therewith; (iii) using the computing device, isolating or extracting ventricular signals from the at least some of the recorded or acquired intra-cardiac signals; and (iv) using the computing device, determining, using the extracted or isolated ventricular signals and the computing device, the locations of the electrodes inside or near the patient's atrium, heart chamber or other portion of the patient's heart that are associated with each isolated or extracted ventricular signal.

In the foregoing system, one or more of the following may be included in the system: (i) configuring the system to navigate the intra-cardiac EP mapping basket of the EP mapping catheter inside or near the atrium or other heart chamber or portion of the patient's heart using the determined locations of the electrodes; (ii) wherein the isolated or extracted ventricular signals comprise at least one of QRS and QRST signals; (iii) wherein the isolated or extracted ventricular signals comprise QRS complexes; (iv) wherein for each electrode an averaged ventricular signal is generated using the plurality of intra-cardiac signals; (v) wherein the averaged ventricular signal is an averaged QRS complex; (vi) configuring the computing device to determine changes in the three-dimensional locations and orientations of the basket and the electrodes thereof as the basket is moved around, in or near the patient's atrium; (vii) configuring the computing device to use position change detection methods to detect basket catheter movements; (viii) configuring the computing device to determine three-dimensional coordinates ***X*** of each electrode for a given position and orientation of the basket within the patient's atrium; (ix) configuring the computing device to embed multiple QRS complexes associated with the ventricular signals acquired or recorded at multiple positions into an embedding space such that multiple positions of the basket at different locations and/or orientations in or near the patient's atrium can be visualized by a user on the screen or monitor; (x) wherein multiple positions of the basket can be employed by the user to navigate the basket inside or near the patient's atrium; (xi) the monitor or screen being configured to provide a visual display to the user of the locations of at least one of the electrodes and the basket inside or near the patient's atrium; (xii) wherein an autoencoder is employed in the computing device to compress QRS complexes into low-dimensional representations thereof; (xiii) wherein the computing device is configured to learn relative positioning of QRS complexes in the embedding space; (xiv) configuring the computing device and the isolated or extracted ventricular signals to employ at least one artificial neural network to determine the locations of at least one of the electrodes and the basket inside or near the patient's atrium; (xv) wherein the at least one artificial neural network further comprises at least one feed forward network; (xvi) wherein the at least one artificial neural network further comprises at least one convolutional neural network; (xvii) wherein the at least one artificial neural network further comprises at least one autoencoder; and (xviii) wherein the locations of the electrodes and/or basket inside or near the patient's atrium are provided in a visualization to a user in real time or near real time.

Further embodiments are disclosed herein or will become apparent to those skilled in the art after having read and understood the claims, specification and drawings hereof.

It will now be seen that the various systems, devices, components and methods disclosed and described herein are capable of detecting with considerable accuracy and precision the locations and types of sources of cardiac rhythm disorders in a patient's heart, diagnosing same, and making better informed and more accurate and likely-to-succeed treatment decisions for patients.

In view of the structural and functional descriptions provided herein, those skilled in the art will appreciate that portions of the described devices and methods may be configured as methods, data processing systems, or computer methods. Accordingly, these portions of the devices and methods described herein may take the form of a hardware embodiment, a software embodiment, or an embodiment combining software and hardware, such as shown and described with respect to computer system 300 illustrated in Fig. 1B. Furthermore, portions of the devices and methods described herein may be a computer method stored in a computer-usable storage medium having computer readable program code on the medium. Any suitable computer-readable medium may be utilized including, but not limited to, static and dynamic storage devices, hard disks, optical storage devices, and magnetic storage devices.

Certain embodiments of portions of the devices and methods described herein are also described with reference to block diagrams of methods, systems, and computer methods. It will be understood that such block diagrams, and combinations of blocks diagrams in the Figures, can be implemented using computer-executable instructions. These computer-executable instructions may be provided to one or more processors of a general purpose computer, a special purpose computer, or any other suitable programmable data processing apparatus (or a combination of devices and circuits) to produce a machine, such that the instructions, which executed via the processor(s), implement the functions specified in the block or blocks of the block diagrams.

These computer-executable instructions may also be stored in a computer-readable memory that can direct computer 300 or other programmable data processing apparatus to function in a particular manner, such that the instructions stored in the computer-readable memory result in an article of manufacture including instructions which implement the function specified in an individual block, plurality of blocks, or block diagram. The computer program instructions may also be loaded onto computer 300 or other programmable data processing apparatus to cause a series of operational steps to be performed on the computer or other programmable apparatus to produce a computer implemented process such that the instructions which execute on computer 300 or other programmable apparatus provide steps for implementing the functions specified in an individual block, plurality of blocks, or block diagram.

In this regard, Fig. 1B illustrates only one example of a computer system 300 (which, by way of example, can include multiple computers or computer workstations) that can be employed to execute one or more embodiments of the devices and methods described and disclosed herein, such as devices and methods configured to acquire and process sensor or electrode data, to process image data, and/or transform sensor or electrode data and image data associated with the analysis of cardiac electrical activity and the carrying out of the combined electrophysiological mapping and analysis of the patient's heart 10 and ablation therapy delivered thereto. Likewise, systems 100 shown in Figs. 2A and 2B may be modified to permit the acquisition of both body surface and intra-cardiac electrode data simultaneously or sequentially.

It will now be seen that the various systems, devices, components and methods disclosed and described herein are capable of detecting with considerable accuracy and precision the locations of sources of cardiac rhythm disorders in a patient's heart.

What have been described above are examples and embodiments of the devices and methods described and disclosed herein. It is, of course, not possible to describe every conceivable combination of components or methodologies for purposes of describing the invention, but one of ordinary skill in the art will recognize that many further combinations and permutations of the devices and methods described and disclosed herein are possible. Accordingly, the devices and methods described and disclosed herein are intended to embrace all such alterations, modifications and variations that fall within the scope of the appended claims. In the claims, unless otherwise indicated, the article "a" is to refer to "one or more than one."

The foregoing outlines features of several embodiments so that those skilled in the art may better understand the detailed description set forth herein. Those skilled in the art will now understand that many different permutations, combinations and variations of the systems, devices, components and methods described and disclosed herein fall within the scope of the various embodiments. Those skilled in the art should appreciate that they may readily use the present disclosure as a basis for designing or modifying other processes and structures for carrying out the same purposes and/or achieving the same advantages of the embodiments introduced herein. Those skilled in the art should also realize that such equivalent constructions do not depart from the spirit and scope of the present disclosure, and that they may make various changes, substitutions and alterations herein without departing from the spirit and scope of the present disclosure.

After having read and understood the present specification, those skilled in the art will now understand and appreciate that the various embodiments described herein provide solutions to long-standing problems, both in the use of electrophysiological mapping systems and in the use of cardiac ablation systems.
The subject matter of the present application relates to, inter alia the following aspects:
1. A method of determining at least one of a position and an orientation of an intra-cardiac electrophysiological **(EP)** mapping basket of an **EP** mapping catheter when the basket is disposed inside or near an atrium or other heart chamber or portion of a patient's heart, the **EP** mapping basket comprising a plurality of electrodes mounted on one or more arms or splines thereof, each electrode having a location or position on one or more splines or arms associated therewith,
   the method comprising:
   recording or acquiring a plurality of intra-cardiac signals inside or near the heart of the patient using the plurality of electrodes, a data acquisition or recording amplifier or device, and a computing device, the data acquisition or recording amplifier or device, and the computing device,
      being operably connected to the **EP** mapping catheter and the electrodes thereof, each of the recorded or acquired intra-cardiac signals having at least one electrode associated therewith;
   using the computing device, isolating or extracting ventricular signals from the at least some of the recorded or acquired intra-cardiac signals;
   using the computing device, determining, using the extracted or isolated ventricular signals and the computing device, the locations of the electrodes inside or near the patient's atrium, heart chamber or other portion of the patient's heart that are associated with each isolated or extracted ventricular signal.
2. The method of aspect 1, further comprising navigating the intra-cardiac **EP** mapping basket of the **EP** mapping catheter inside or near the atrium or other heart chamber or portion of the patient's heart using the determined locations of the electrodes.
3. The method of aspect 1 or 2, wherein the isolated or extracted ventricular signals comprise at least one of QRS and QRST signals.
4. The method of aspect 3, further comprising recording or acquiring at least one body surface signal from the patient using at least one body surface electrode, the at least one body surface signal being acquired or recorded simultaneously or substantially simultaneously with the recorded or acquired intra-cardiac signals.
5. The method of aspect 4, further comprising, using the computing device, isolating or extracting at least one ventricular signal from the recorded or acquired body surface signal, and determining, using the extracted or isolated intra-cardiac and at least one body surface ventricular signals and the computing device, the locations of the electrodes inside the patient's heart that are associated with each isolated or extracted ventricular signal.
6. The method of one of aspects 1 to 5, further comprising moving the basket inside the patient's atrium to different positions, and recording or acquiring intra-cardiac signals at each such position.
7. The method of one of aspects 1 to 6, further comprising, using the computing device, determining changes in the three-dimensional locations and orientations of the basket and the electrodes thereof as the basket is moved around, in or near the patient's atrium.
8. The method of one of aspects 1 to 7, further comprising, using the computing device, determining the three-dimensional coordinates *X* of each electrode for a given position and orientation of the basket within the patient's atrium.
9. A system configured to determine at least one of a position and an orientation of an intra-cardiac electrophysiological **(EP)** mapping basket of an **EP** mapping catheter when the basket is disposed inside or near an atrium or other heart chamber or portion of a patient's heart, the **EP** mapping basket comprising a plurality of electrodes mounted on one or more arms or splines thereof, each electrode having a location or position on one or more splines or arms associated therewith, the system comprising:
   (a) at least one computing device;
   (b) at least one data acquisition device operably connected to the at least one computing device or configured to provide as outputs therefrom at least one of electrical signals and at least one body surface electrogram signal;
   (c) a display or monitor operably connected to the at least one computing device and configured to visually display to a user results generated by
   the at least one computing device;
   wherein the computing device comprises at least one non-transitory computer readable medium configured to store instructions executable by at least one processor to extract atrial signals from the plurality of electrical signals, the computing device being configured to: (i) receive or acquire, using the data acquisition device, the plurality of electrical signals using electrodes and at least one body surface electrogram signal using the at least one body surface electrode located on one or more body surfaces of the patient, wherein the electrical signals and the at least one body surface electrogram signal are acquired over the same or substantially the same first time window, the first time window being sufficiently long to capture a plurality of QRS complexes; (ii) recording or acquiring a plurality of intra-cardiac signals inside or near the heart of the patient using the plurality of electrodes, the data acquisition device, and the computing device, the data acquisition device and the computing device being operably connected to the **EP** mapping catheter and the electrodes thereof, each of the recorded or acquired intra-cardiac signals having at least one electrode associated therewith; (iii) using the computing device, isolating or extracting ventricular signals from the at least some of the recorded or acquired intra-cardiac signals; and (iv) using the computing device, determining, using the extracted or isolated ventricular signals and the computing device, the locations of the electrodes inside or near the patient's atrium, heart chamber or other portion of the patient's heart that are associated with each isolated or extracted ventricular signal.
10. The system of aspect 9, further comprising configuring the system to navigate the intra-cardiac **EP** mapping basket of the **EP** mapping catheter inside or near the atrium or other heart chamber or portion of the patient's heart using the determined locations of the electrodes.
11. The system of aspect 9 or 10, wherein the isolated or extracted ventricular signals comprise at least one of QRS and QRST signals.
12. The system of one of aspects 9 to 11, further comprising configuring the computing device to determine changes in the three-dimensional locations and orientations of the basket and the electrodes thereof as the basket is moved around, in or near the patient's atrium.
13. The system of one of aspects 9 to 12, further comprising configuring the computing device to use position change detection methods to detect basket catheter movements.
14. The system of one of aspects 9 to 13, further comprising configuring the computing device to determine three-dimensional coordinates X of each electrode for a given position and orientation of the basket within the patient's atrium.

## Claims

1. A system for determining at least one of a position and an orientation of an intra-cardiac electrophysiological (EP) mapping basket of an EP mapping catheter when the basket is disposed inside or near an atrium or other heart chamber or portion of a patient's heart, the system comprising:
an EP mapping catheter having an EP mapping basket, the EP mapping basket comprising a plurality of electrodes mounted on one or more arms or splines thereof, each electrode having a location or position on one or more splines or arms associated therewith;
a data acquisition or recording amplifier or device; and
a computing device,
wherein the data acquisition or recording amplifier or device and the computing device are operably connected to the EP mapping basket catheter and the electrodes thereof and are configured to record or acquire a plurality of intra-cardiac signals inside or near the heart of the patient using the plurality of electrodes when the basket is disposed inside or near an atrium or other heart chamber or portion of a patient's heart, each of the recorded or acquired intra-cardiac signals having at least one electrode of the plurality of electrodes associated therewith, and
wherein the computing device is further configured to:
isolate or extract ventricular signals from the at least some of the recorded or acquired intra-cardiac signals; and
determine, using the extracted or isolated ventricular signals, the locations of the electrodes inside or near the patient's atrium, heart chamber or other portion of the patient's heart that are associated with each isolated or extracted ventricular signal.

2. The system of claim 1, wherein the isolated or extracted ventricular signals comprise at least one of QRS and QRST signals.

3. The system of claim 2, further comprising at least one body surface electrode, and wherein the data acquisition or recording amplifier or device and the computing device are operably connected to the at least one body surface electrode and are configured to record or acquire at least one body surface signal from the patient using the at least one body surface electrode, the at least one body surface signal being acquired or recorded simultaneously or substantially simultaneously with the recorded or acquired intra-cardiac signals.

4. The system of claim 3, wherein the computing device is further configured to isolate or extract at least one ventricular signal from the recorded or acquired body surface signal, and determine, using the extracted or isolated intra-cardiac and at least one body surface ventricular signals, the locations of the electrodes inside the patient's heart that are associated with each isolated or extracted ventricular signal.

5. The system of one of claims 1 to 4, wherein the computing device is further configured to determine changes in the three-dimensional locations and orientations of the basket and the electrodes thereof as the basket is moved around, in or near the patient's atrium.

6. The system of any of claims 1 to 5, wherein the computing device is further configured to determine the three-dimensional coordinates ***X*** of each electrode for a given position and orientation of the basket within the patient's atrium.
